(19) Europäisches Patentamt
European Patent Office
Office européen des brevets

(11) EP 1 095 653 A1

(12) **EUROPEAN PATENT APPLICATION**
published in accordance with Art. 158(3) EPC

(43) Date of publication:
**02.05.2001 Bulletin 2001/18**

(21) Application number: **99925324.8**

(22) Date of filing: **15.06.1999**

(51) Int. Cl.7: **A61K 31/215**, A61K 31/70, A61K 31/35, C07D 309/14, C07C 305/20, C07H 13/04

(86) International application number:
**PCT/JP99/03180**

(87) International publication number:
**WO 99/65480 (23.12.1999 Gazette 1999/51)**

(84) Designated Contracting States:
**AT BE CH DE DK ES FI FR GB GR IE IT LI LU NL PT SE**

(30) Priority: **16.06.1998 JP 18327698**

(71) Applicant:
**ONO PHARMACEUTICAL CO., LTD.**
**Osaka-shi, Osaka 541-8526 (JP)**

(72) Inventors:
- **FUKUSHIMA, Daikichi,**
**Minase Research Institute**
**Mishima-gun, Osaka 618-8585 (JP)**
- **SHIBAYAMA, Shiro,**
**Minase Research Institute**
**Mishima-gun, Osaka 618-8585 (JP)**
- **TADA, Hideaki,**
**Minase Research Institute**
**Mishima-gun, Osaka 618-8585 (JP)**

(74) Representative:
**Henkel, Feiler, Hänzel**
**Möhlstrasse 37**
**81675 München (DE)**

# (54) GLUCOPYRANOSE DERIVATIVES AND PREVENTIVES AND/OR REMEDIES FOR HIV INFECTION CONTAINING THE SAME AS THE ACTIVE INGREDIENT

(57) The present invention relates to a medicament for preventing and / or treating HIV infectious diseases comprising a compound of formula (J) as active ingredient and glucopyranose derivative of formula (W) or non-toxic salts thereof (the symbols in the formula are as described in the specification).

A glucopyranose derivative of formulae (J) or (W) or a non-toxic salt thereof is useful as a medicament for preventing and / or treating HIV infectious diseases (AIDS).

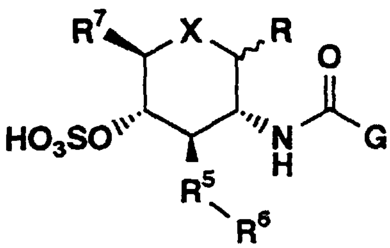

(J)

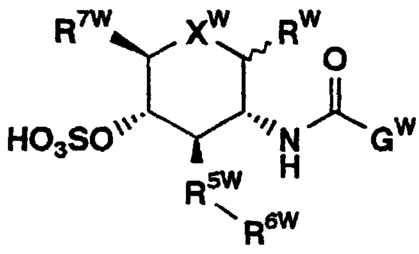

(W)

EP 1 095 653 A1

## Description

Technical Field

The present invention relates to:

(1) a medicament for preventing and/or treating HIV infection diseases comprising, as an active ingredient, a glucopyranose derivative of formula (J):

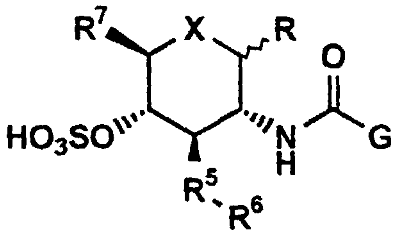

(J)

(wherein all the symbols have the same meanings as described below), or a non-toxic salt thereof;
(2) a glucopyranose derivative of formula (W):

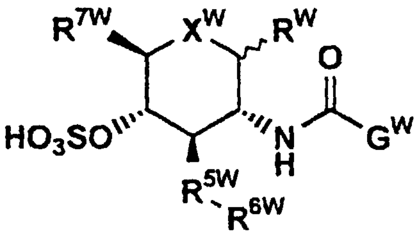

(W)

(wherein all the symbols have the same meanings as described below), or a non-toxic salt thereof;
(3) a method for production thereof; and
(4) a medicament for preventing and/or treating HIV infection diseases comprising those compounds as active ingredients.

Background of the Invention

Acquired immune deficiency syndrome (hereinafter referred to as "AIDS") caused by the infection of human immunodeficiency virus (hereinafter referred to as "HIV") is one of the diseases whose methods for treatment are recently most desired earnestly. When the infection of HIV to CD4 positive cells is once established, HIV repeats multiplication in a patient's body, and then fatally destroys T cell which takes charge of the immunological function. During the process, immunological function is gradually deteriorated to result in the appearance of various immunologically deficient states such as pyrexia, diarrhea and swelling of lymph nodes, which, in the course of time, tend to be complicated by various opportunistic infections such as Kalini pneumonia. Such a situation is regarded as occurrence of AIDS, and it is well known that Kaposi's sarcoma is induced and the patient falls into a critical condition. Currently, a variety of methods for prevention and treatment have been attempted against AIDS (for example, (1) inhibition of the multiplication of HIV by administering a reverse transcriptase inhibitor or a protease inhibitor, (2) prevention or alleviation of opportunistic infections by administering a medicament having immunopotentiating activity).

The present inventors have focused their attention to the fact that HIV binds to T cell through a membrane protein and invades therein, and have searched for a possibility to prevent and/or treat acquired immune deficiency syndrome (AIDS) caused by the infection of HIV based on the following mechanisms.

HIV infects mainly helper T cell which takes charge of the center of immune system. It has been known since 1985 that HIV utilizes a protein molecule, CD4 expressed on the membrane of T cell, at that time (*Cell*, 52, 631 (1985)). A CD4 molecule is composed of 433 amino acid residues, and expressed in macrophages, part of B cells, angioendothelial cells, Langerhans cells in skin tissues, dendritic cells in lymphoid tissues, glia cells at central nervous system and the like other than mature helper T calls. However, with the discovery that the infection of HIV is not established by

the CD4 molecule alone, a possibility is suggested that a factor participating the infection of HIV to a cell exists other than the CD4 molecule. In 1996, a cell membrane protein named Fusin was identified as a factor necessary for the infection of HIV to a cell other than the CD4 molecule (*Science*, 272, 872 (1996)). Before long, it was proved that the Fusin molecule was a receptor of stromal derived factor-1 (abbreviated as SDF-1), i.e., CXCR4 and also SDF-1 specifically suppresses the infection with T cell-directing HIV *in vitro* (Nature, 382, 829 (1996), *Nature*, 382, 833 (1996)). Namely, it is considered that the infection of HIV is inhibited by taking a foothold for the infection of HIV to a cell through the binding of SDF-1 with CXCR4 in advance. Therefore, it is expected that a substance capable of taking CXCR4 in competition with HIV or making HIV impossible to bind to CXCR4 through binding to the virus may be an inhibitor for the infection of HIV. In addition, there is an example that a low molecular-weight compound discovered as an inhibitor for the infection of HIV at first is later confirmed as an antagonist of CXCR4 (*Nature Medicine*, 4, 72 (1998)).

As a result of studies of searching for a compound of a possible inhibitor for the infection of HIV based on these aspects, the present inventors have found that a glucopyranose derivative achieves the object of the present invention, and have accomplished the present invention.

Among the glucopyranose derivatives of formula (I):

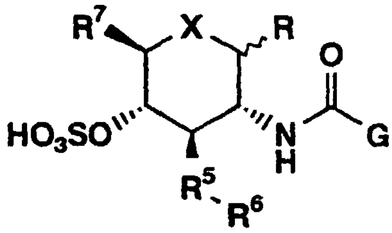

(I)

(wherein all the symbols have the same meanings as described below) or a non-toxic salt thereof, it is disclosed that the following compounds have a lipid A-like activity and have immunopotentiating activity (such as macrophage-activating activity, B cell-blastogenesis activity, non-specific antibody-producing activity, cellular immunopotentiating activity, and the like) and antitumor activity (such as interferon-inducing activity, interleukine-producing activity, TNF-inducing activity, and the like):

1) a glucopyranose derivative of formula (I-A):

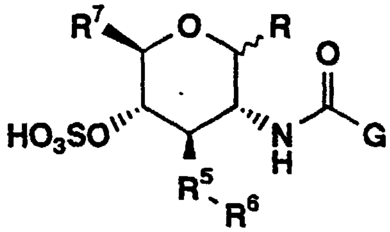

(I-A)

(wherein all the symbols have the same meanings as described below) or a non-toxic salt thereof, in JP-B-4-74359 (EP 226381) and Japanese Patent 2514070 (EP 288888); and

2) a cyclohexane derivative of formula (I-C):

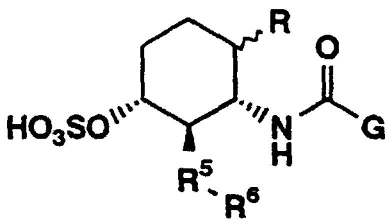

(I-C)

(wherein all the symbols have the same meanings as described below) or a non-toxic salt thereof in JP-A-63-297357.

Moreover, among the compounds of formula (I), various non-toxic salts of 2-deoxy-2-[3S-(9-phenylnonanoyloxy)tetradecanoyl]amino-3-O-(9-phenylnonanoyl)-4-O-sulfo-D-glucopyranose of formula (I-a) are disclosed in JP-A-6-41175 (EP 553786).

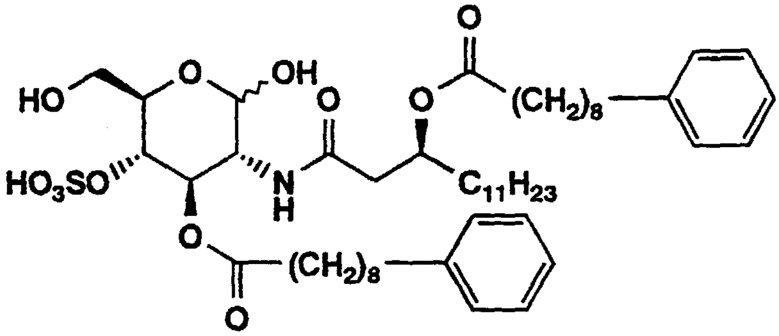

(I-a)

Furthermore, JP-W-60-501259 (EP 143840) describes that a pharmaceutical composition comprising, together with a pharmaceutical carrier, a compound of formula (T):

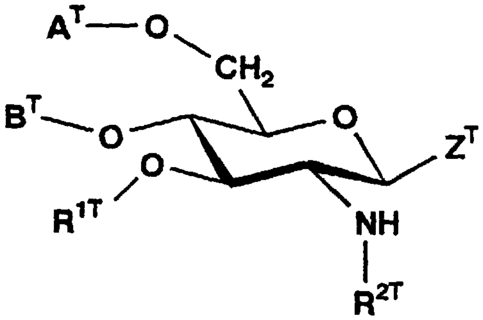

(T)

(wherein a preferred stereochemical structure is that of glucosamine; $A^T$ and $B^T$ are the same or different and each represents H, a hydrocarbon structure, a fatty acid acyl chain, or another functional group; $R^{1T}$ and $R^{2T}$ are the same or different and each represents H, a hydrocarbon structure, or a fatty acid acyl chain; when a hydroxyl substituent is present on $A^T$, $B^T$, $R^{1T}$, and/or $R^{2T}$, this can be further substituted with a fatty acid acyl chain; and the substituent $Z^T$ is a group imparting solubility in water).

However, it is not reported at all that a glucopyranose derivative of formula (I) or (T), or a non-toxic salt thereof is effective as a medicament for prevention and/or treatment of HIV infectious diseases (AIDS).

Disclosure of the Invention

The present invention relates to:

1) a medicament for preventing and/or treating HIV infectious diseases comprising, as an active ingredient, a glucopyranose derivative of formula (J):

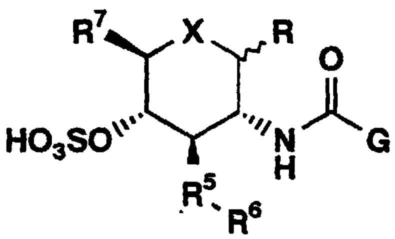

(J)

(wherein X represents an oxygen atom or a methylene group;
R represents a hydrogen atom, a hydroxyl group, or a C1-4 alkoxy group;
G represents:

(1) a group of formula:

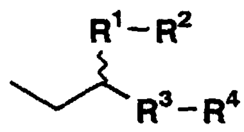

(wherein $R^1$ represents a single bond or a C2-20 oxycarbonylalkylene group;
$R^2$ represents hydrogen atom or a group of formula:

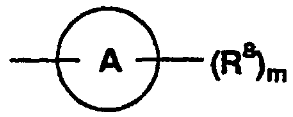
or
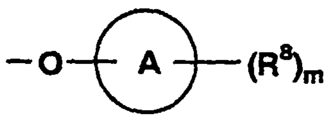

(in each formula, the ring A represents a C5-15 carbon ring; $R^8$ represents a hydrogen atom, a C1-7 alkyl group, a C1-7 alkoxy group, or a halogen atom; and m represents 1, 2 or 3);
$R^3$ represents a C1-20 alkylene group; and
$R^4$ represents a hydrogen atom or a group of formula:

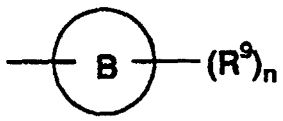
or
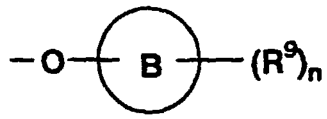

(in each formula, the ring B represents a C5-15 carbon ring; $R^9$ represents a hydrogen atom, a C1-7 alkyl group, a C1-7 alkoxy group, or a halogen atom; and n represents 1, 2 or 3)), or

(2) a group of formula:

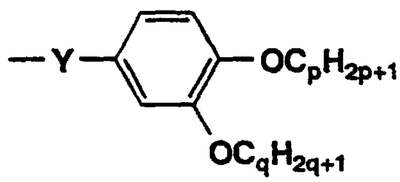

(wherein Y represents a single bond or a C1-4 alkylene group; and p and q each independently represents an integer of 6 to 12);
$R^5$ represents a C2-20 oxycarbonylalkylene group;
$R^6$ represents a hydrogen atom or a group of formula:

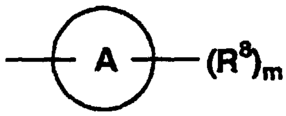

or

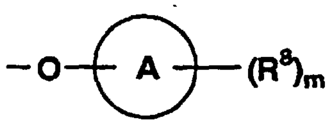

(wherein the ring A, $R^8$ and m have the same meanings as described above), or a combination of $R^5$-$R^6$ represents a group of formula:

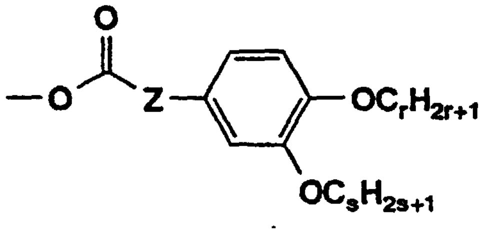

(wherein Z represents a single bond or a C1-4 alkylene group; and r and s each independently represents an integer of 6 to 12); and
$R^7$ represents a hydrogen atom, a methyl group, a hydroxymethyl group, or a sulfoxymethyl group;
with the proviso that

(1) when $R^1$ represents a C2-20 oxycarbonylalkylene group, $R^2$ is bound to the alkyl group in $R^1$,
(2) when $R^5$ represents a C2-20 oxycarbonylalkylene group, $R^6$ is bound to the alkyl group in $R^5$),

or a non-toxic salt thereof;

2) a medicament for preventing and/or treating HIV infectious diseases comprising, as an active ingredient, a glucopyranose derivative of formula (I):

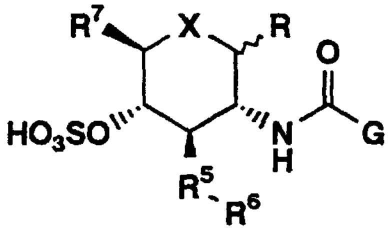

(I)

(wherein X represents an oxygen atom or a methylene group;
R represents a hydrogen atom, a hydroxyl group, or a C1-4 alkoxy group;
G represents:

(1) a group of formula:

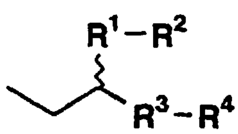

(wherein $R^1$ represents a single bond or a C2-20 oxycarbonylalkylene group;
$R^2$ represents a hydrogen atom or a group of formula:

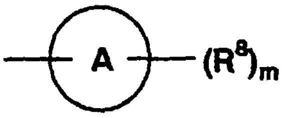

or

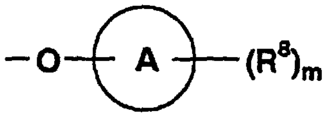

(in each formula, the ring A represents a C5-15 carbon ring; $R^8$ represents hydrogen atom, a C1-7 alkyl group, a C1-7 alkoxy group, or a halogen atom; and m represents 1, 2 or 3);
$R^3$ represents a C1-20 alkylene group; and
$R^4$ represents a hydrogen atom or a group of formula:

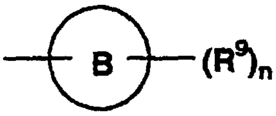

or

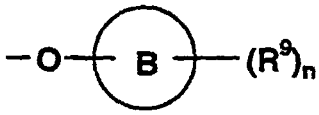

(in each formula, the ring B represents a C5-15 carbon ring; $R^9$ represents a hydrogen atom, a C1-7 alkyl group, a C1-7 alkoxy group, or a halogen atom; and n represents 1, 2 or 3)), or

(2) a group of formula:

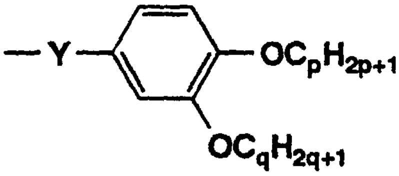

(wherein Y represents a single bond or a C1-4 alkylene group; and p and q each independently represents an integer of 6 to 12);
$R^5$ represents a C2-20 oxycarbonylalkylene group;
$R^6$ represents hydrogen atom or a group of the formula:

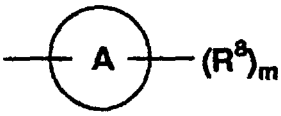

or

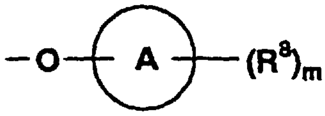

(wherein the ring A, $R^8$ and m have the same meanings as described above), or a combination of $R^5$-$R^6$ represents a group of formula:

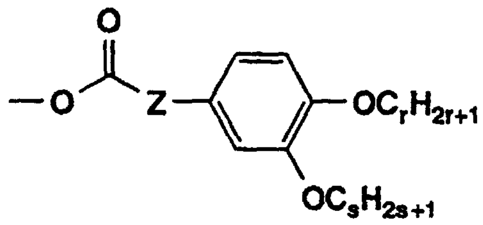

(wherein Z represents a single bond or a C1-4 alkylene group and r and s each independently represents an integer of 6 to 12);
$R^7$ represents a hydrogen atom, a methyl group, a hydroxymethyl group, or a sulfoxymethyl group; with the proviso that

(1) when $R^1$ represents a C2-20 oxycarbonylalkylene group, $R^2$ is bound to the alkyl group in $R^1$,
(2) when $R^5$ represents a C2-20 oxycarbonylalkylene group, $R^6$ is bound to the alkyl group in $R^5$), and
(3) when $R^7$ represents a methyl group, a hydroxymethyl group, or a sulfoxymethyl group, $R^2$, $R^4$ and $R^6$ do not represent hydrogen atoms at the same time),

or a non-toxic salt thereof as an active ingredient;

3) a glucopyranose derivative of formula (W):

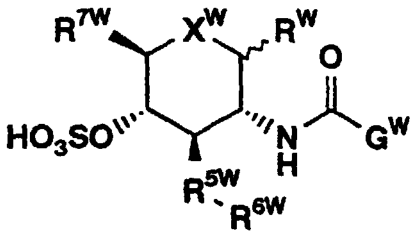

(W)

(wherein $X^W$ represents an oxygen atom or a methylene group;
$R^W$ represents a hydrogen atom, a hydroxyl group, or a C1-4 alkoxy group;
$G^W$ represents:

(1) a group of formula:

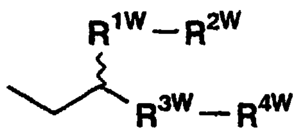

(wherein $R^{1W}$ represents a single bond or a C2-20 oxycarbonylalkylene group;
$R^{2W}$ represents a hydrogen atom;
$R^{3W}$ represents a C1-20 alkylene group;
$R^{4W}$ represents a hydrogen atom;
$R^{5W}$ represents a C2-20 oxycarbonylalkylene group;
$R^{6W}$ represents a hydrogen atom; and

$R^{7W}$ represents a methyl group, a hydroxymethyl group, or a sulfoxymethyl group;
with the proviso that

(1) when $R^{1W}$ represents a C2-20 oxycarbonylalkylene group, $R^{2W}$ is bound to the alkyl group in $R^{1W}$, and
(2) when $R^{5W}$ represents a C2-20 oxycarbonylalkylene group, $R^{6W}$ is bound to the alkyl group in $R^{5W}$),

or a non-toxic salt thereof;

4) a method for preparation of a glucopyranose derivative of formula (W) or a non-toxic salt thereof; and

5) a medicament for preventing and/or treating HIV infectious diseases comprising, as an active ingredient, a glucopyranose derivative of formula (W) or a non-toxic salt thereof.

Brief Description of the Drawings

Fig. 1 is a graph showing an effect of the compound of the present invention on inhibition against HIV IIIB virus infection.
Fig. 2 is a graph showing cytotoxicity of the compound of the present invention against MTS cells.

Details of the Present Invention

When X represents an oxygen atom in formula (I), it corresponds to a glucopyranose derivative of formula (I-A):

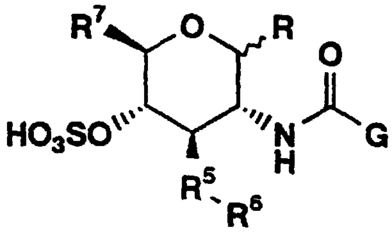

(I-A)

(wherein all the symbols have the same meanings as described above). In this case, $R^7$ is preferably any of the groups but is more preferably a hydroxymethyl group.

On the other hand, when X represents a methylene group, it corresponds to a cyclohexane derivative of formula (I-B):

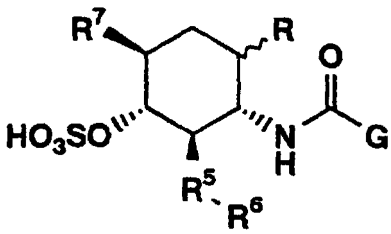

(I-B)

(wherein all the symbols have the same meanings as described above). In this case, $R^7$ is preferably any of the groups but is more preferably hydrogen atom, i.e., a cyclohexane derivative of formula (I-C):

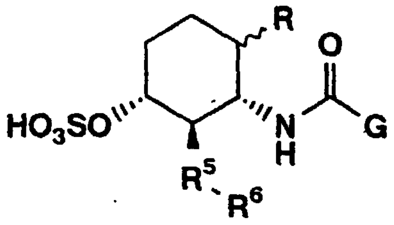

(I-C)

(wherein all the symbols have the same meanings as described above).

When $X^W$ represents oxygen atom in formula (W), it corresponds to a glucopyranose derivative of formula (W-A):

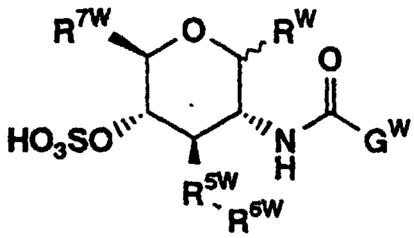

(W-A)

(wherein all the symbols have the same meanings as described above). In this case, $R^{7W}$ is preferably any of the groups but is more preferably hydroxymethyl group.

On the other hand, when $X^W$ represents a methylene group, it corresponds to a cyclohexane derivative of formula (W-B):

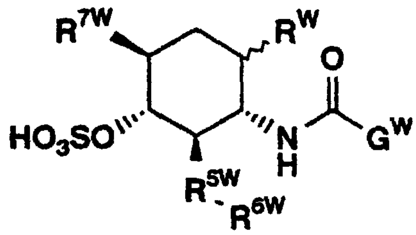

(W-B)

(wherein all the symbols have the same meanings as described above). In this case, $R^{7W}$ is preferably any of the groups but is more preferably hydroxymethyl group.

In formulae (J) and (I), examples of the C1-4 alkoxy group which R represents include methoxy, ethoxy, propoxy and butoxy groups, and isomers thereof. Preferred examples of R include a hydrogen atom, a hydroxyl group and a methoxy group.

In formulae (J) and (I), the C2-20 oxycarbonylalkylene group which $R^1$ and $R^5$ in G represent is of formula:

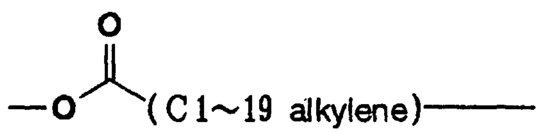

wherein the side chain of the alkylene group in the formula is bound to $R^2$ or $R^6$. In the formula, examples of the C1-19 alkylene group include methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene, and nonadecamethylene groups, and isomers thereof.

Preferred examples of $R^1$ include a single bond (in this case, $R^2$ is preferably a hydrogen atom), oxycarbonylhexamethylene, oxycarbonylheptamethylene, oxycarbonyloctamethylene, oxycarbonylnonamethylene, and oxycarbonyldecamethylene groups; and a single bond (in this case, $R^2$ is preferably a hydrogen atom) and an oxycarbonyloctamethylene group are more preferred.

Preferred examples of the C2-20 oxycarbonylalkylene group of $R^5$ include oxycarbonylhexamethylene, oxycarbonylheptamethylene, oxycarbonyloctamethylene, oxycarbonylnonamethylene, and oxycarbonyldecamethylene groups; and an oxycarbonyloctamethylene group is more preferred.

In formulae (J) and (I), the C5-15 carbon ring represented by the ring A in $R^2$ and $R^6$ in G and the ring B in $R^4$ in G means a C5-15 monocyclic, bicyclic or tricyclic aromatic carbon ring, a partially saturated carbon ring thereof, or a completely saturated carbon ring thereof.

Examples of the above-described C5-15 monocyclic, bicyclic or tricyclic aromatic carbon ring include benzene, naphthalene, indene, fluorene, and anthracene rings.

Examples of the above-described partially saturated carbon ring of the C5-15 monocyclic, bicyclic or tricyclic aromatic carbon ring include cyclopentene, cyclohexene, cyclohexadiene, cycloheptene, cycloheptadiene, 1,2,3,4-tetrahydronaphthalene, indane, and 1,2,3,4-tetrahydrofluorene rings, and the like.

Examples of the above-described completely saturated carbon ring of the C5-15 monocyclic, bicyclic or tricyclic aromatic carbon ring include C5-7 cycloalkyl rings, such as cyclopentyl, cyclohexyl and cycloheptyl rings, and decahydronaphthalene and decahydrofluorene rings.

Preferred examples of the ring A and ring B include C5-10 monocyclic or bicyclic aromatic carbon rings; benzene and naphthalene rings are more preferred; and a benzene ring is more preferred.

In formulae (J) and (I), examples of the C1-7 alkyl group represented by $R^8$ in $R^2$ and $R^6$ in G and $R^9$ in $R^4$ in G include methyl, ethyl, propyl, butyl, pentyl, hexyl, and heptyl groups, and isomers thereof.

In formulae (J) and (I), examples of the C1-7 alkoxy group represented by the above-described $R^8$ and $R^9$ include methoxy, ethoxy, propoxy, butoxy, pentyloxy, hexyloxy, and heptyloxy groups, and isomers thereof.

In formulae (J) and (I), examples of the halogen atom represented by the above-described $R^8$ and $R^9$ include chlorine, bromine, fluorine, and iodine atoms.

Preferred examples of $R^8$ and $R^9$ include a hydrogen atom, a pentyl group, a methoxy group, and a chlorine atom, and a hydrogen atom is more preferred.

In formulae (J) and (I), examples of the C1-20 alkylene group represented by $R^3$ include methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene, nonadecamethylene, and eicosamethylene groups, and isomers thereof.

Preferred examples of $R^3$ include nonamethylene, decamethylene, undecamethylene, dodecamethylene, and tridecamethylene groups, and an undecamethylene group is more preferred.

In formulae (J) and (I), examples of the C1-4 alkylene group represented by Y in G and Z in the group represented by $R^5$-$R^6$ include methylene, ethylene, trimethylene, and tetramethylene groups, and isomers thereof. A preferred example of Y and Z includes a single bond.

In formulae (J) and (I), preferred examples of p and q in G and r and s in the group represented by $R^5$-$R^6$ include 8, 9, 10 and 11, and 8 and 10 are more preferred.

In formula (W), examples of the C1-4 alkoxy group represented by $R^W$ include methoxy, ethoxy, propoxy, and butoxy groups, and isomers thereof. Preferred examples of $R^W$ include a hydrogen atom, a hydroxyl group, and a methoxy group.

In formula (W), the C2-20 oxycarbonylalkylene group represented by $R^{1W}$ and $R^{5W}$ in $G^W$ is represented by formula:

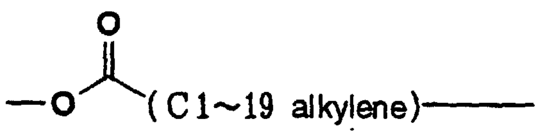

wherein the side chain of the alkylene group in the formula is bound to $R^{2W}$ or $R^{6W}$. in the formula, examples of the C1-19 alkylene group include methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene, and nonadecamethylene groups, and isomers thereof.

Preferred examples of $R^{1W}$ include a single bond and C5-15 oxycarbonylalkylene groups, i.e., oxycarbonylpentamethlene, oxycarbonylhexamethylene, oxycarbonylheptamethylene, oxycarbonyloctamethylene, oxycarbonylnonamethylene, oxycarbonyldecamethylene, oxycarbonylundecamethylene, oxycarbonyldodecamethylene, oxycarbonyltridecamethylene, oxycarbonyltetradecamethylene, and oxycarbonylpentadecamethylene groups; and a single bond and C5-15 oxycarbonylalkylene groups, i.e., oxycarbonylpentamethlene, oxycarbonylnonamethylene, oxycarbonylundecamethylene, and oxycarbonyltridecamethylene groups, are more preferred.

Preferred examples of the C2-20 oxycarbonylalkylene group represented by $R^{5W}$ include C5-15 oxycarbonylalkylene groups, i.e., oxycarbonylpentamethlene, oxycarbonylhexamethylene, oxycarbonylheptamethylene, oxycarbonyloctamethylene, oxycarbonylnonamethylene, oxycarbonyldecamethylene, oxycarbonylundecamethylene, oxycarbonyldodecamethylene, oxycarbonyltridecamethylene, oxycarbonyltetradecamethylene, and oxycarbonylpentadecamethylene groups; and oxycarbonylpentamethlene, oxycarbonylnonamethylene, oxycarbonylundecamethylene, and oxycarbonyltridecamethylene groups are more preferred.

In formula (W), examples of the C1-20 alkylene group represented by $R^{3W}$ include methylene, ethylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, heptadecamethylene, octadecamethylene, nonadecamethylene, and eicosamethylene groups, and isomers thereof.

Preferred examples of $R^{3W}$ include C9-18 alkylene groups, i.e., nonamethylene, decamethylene, undecamethylene, dodecamethylene, tridecamethylene, tetradecamethylene, pentadecamethylene, hexadecamethylene, heptadecamethylene and octadecamethylene groups; and C11-15 alkylene groups, i.e., undecamethylene group, dodecamethylene, tridecamethylene, tetradecamethylene, and pentadecamethylene groups, are more preferred.

In the present invention, unless otherwise mentioned, the symbol 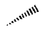 means a bonding toward backside of the paper face, i.e., α-configuration, the symbol 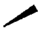 means a bonding toward front side of the paper face, i.e., β-configuration, and the symbol 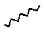 means α-, β- or a mixture thereof.

In the present invention, unless otherwise mentioned, isomers are all included. For example, the alkyl group, the alkoxy group and the alkylene group include straight chain and branched ones. Furthermore, isomers (E, Z, cis, trans isomer) at a double bond, ring and condensed ring, isomers resulted from the presence of an asymmetric carbon (R, S isomers, α, β isomers, enantiomer, diastereomer), optically active compounds having optical rotation (D, L, d, l isomers), polar substances at chromatographic separation (more polar substance, less polar substance), equilibrium compounds, mixtures of any proportion of these compounds, and racemic mixtures are all included in the present invention.

The preferred compounds used in the present invention represented by formula (J), (I), or (W) are the compounds used in the following examples (experimental examples), the compounds described in the examples of JP-B-4-74359 (EP 226381), Japanese Patent No. 2514070 (EP 288888) or JP-A-63-297357, the compounds shown in following Tables 1 to 24, and salts thereof.

In Tables 1 to 24, OMe, Ph, Me and OBu represent a methoxy group, a phenyl group, a methyl group, and a butoxy group, respectively; the preceding number thereof represents a position of each substituent; di and tri represent di-substituted and tri-substituted, respectively; and the various alkoxy groups and alkyl groups are all linear ones.

Furthermore, in Tables 1 to 24,

(R)-$CH_2CH(OCOC_5H_{11})C_{11}H_{23}$ represents

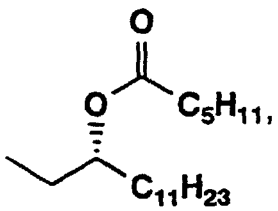

(S)-$CH_2CH(OCOC_5H_{11})C_{11}H_{23}$ represents

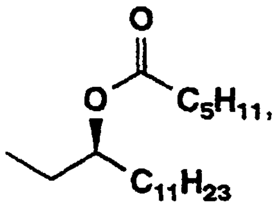

(R)-$CH_2CH(OCOC_9H_{19})C_{11}H_{23}$ represents

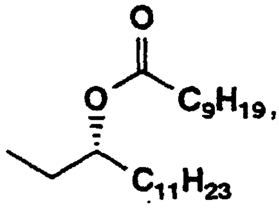

(S)-$CH_2CH(OCOC_9H_{19})C_{11}H_{23}$ represents

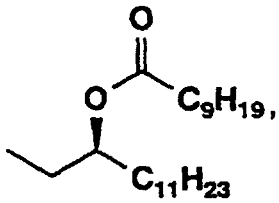

(R)-$CH_2CH(OCOC_{13}H_{27})C_{13}H_{27}$ represents

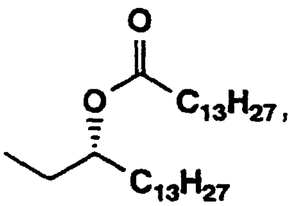

, and

(S)-$CH_2CH(OCOC_{13}H_{27})C_{13}H_{27}$ represents

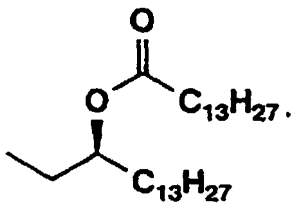

For example, it is meant that each of Tables 1 to 4 contains 867 compounds (i.e., 3 x 17 x 17 = 867) which results from the combinations of the substituents of three kinds as R and seventeen kinds as each of the following groups:

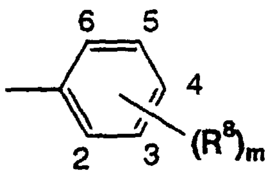 and 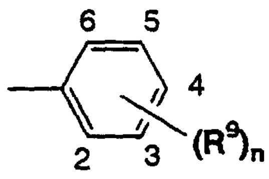

Similarly, it is meant that each of Tables 5 to 8 contains 168 compounds (i.e., 3 x 7 x 8 = 168) which results from the combinations of the substituents of three kinds as R, eight kinds as G and seven kinds as the following group:

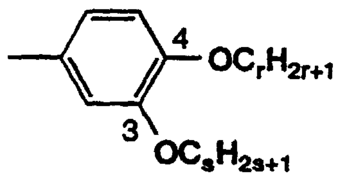

Furthermore, it is meant that each of Tables 9 to 12 contains 315 compounds (i.e., 3 x 15 x 7 = 168) which results from the combinations of the substituents of three kinds as $R^W$, fifteen kinds as $G^W$ and seven kinds as $-R^{5W}-R^{6W}$.

## Table 1

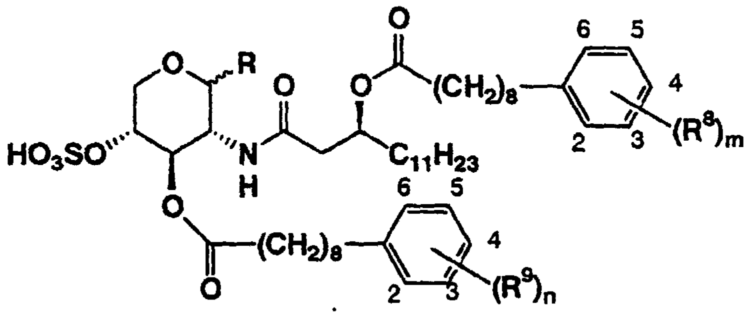

(I-1)

| R | 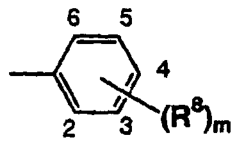 | 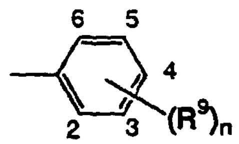 |
|---|---|---|
| (1) H | (1) Ph | (1) Ph |
| (2) OH | (2) 2-Cl-Ph | (2) 2-Cl-Ph |
| (3) OMe | (3) 2-Me-Ph | (3) 2-Me-Ph |
| | (4) 2-OBu-Ph | (4) 2-OBu-Ph |
| | (5) 3-Cl-Ph | (5) 3-Cl-Ph |
| | (6) 3-Me-Ph | (6) 3-Me-Ph |
| | (7) 3-OBu-Ph | (7) 3-OBu-Ph |
| | (8) 4-Cl-Ph | (8) 4-Cl-Ph |
| | (9) 4-Me-Ph | (9) 4-Me-Ph |
| | (10) 4-OBu-Ph | (10) 4-OBu-Ph |
| | (11) 2,4-diCl-Ph | (11) 2,4-diCl-Ph |
| | (12) 3,4-diCl-Ph | (12) 3,4-diCl-Ph |
| | (13) 2,4,6-triCl-Ph | (13) 2,4,6-triCl-Ph |
| | (14) 2,4-diOBu-Ph | (14) 2,4-diOBu-Ph |
| | (15) 3,4-diOBu-Ph | (15) 3,4-diOBu-Ph |
| | (16) 3-Me-4-Cl-Ph | (16) 3-Me-4-Cl-Ph |
| | (17) 3-Cl-5-OBu-Ph | (17) 3-Cl-5-OBu-Ph |

## Table 2

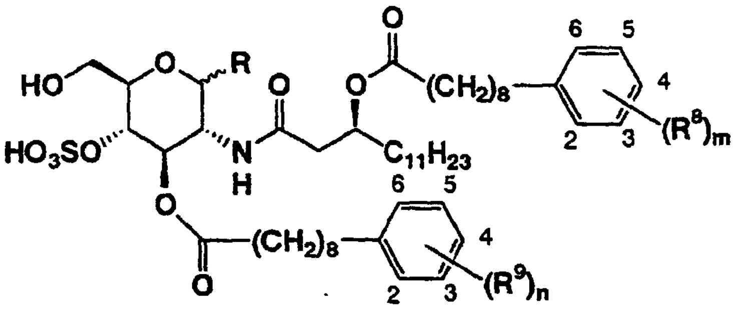

(I-2)

| R | 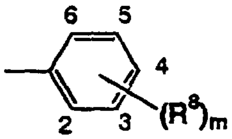 | 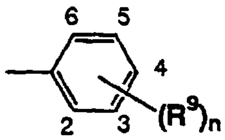 |
|---|---|---|
| (1) H | (1) Ph | (1) Ph |
| (2) OH | (2) 2-Cl-Ph | (2) 2-Cl-Ph |
| (3) OMe | (3) 2-Me-Ph | (3) 2-Me-Ph |
| | (4) 2-OBu-Ph | (4) 2-OBu-Ph |
| | (5) 3-Cl-Ph | (5) 3-Cl-Ph |
| | (6) 3-Me-Ph | (6) 3-Me-Ph |
| | (7) 3-OBu-Ph | (7) 3-OBu-Ph |
| | (8) 4-Cl-Ph | (8) 4-Cl-Ph |
| | (9) 4-Me-Ph | (9) 4-Me-Ph |
| | (10) 4-OBu-Ph | (10) 4-OBu-Ph |
| | (11) 2,4-diCl-Ph | (11) 2,4-diCl-Ph |
| | (12) 3,4-diCl-Ph | (12) 3,4-diCl-Ph |
| | (13) 2,4,6-triCl-Ph | (13) 2,4,6-triCl-Ph |
| | (14) 2,4-diOBu-Ph | (14) 2,4-diOBu-Ph |
| | (15) 3,4-diOBu-Ph | (15) 3,4-diOBu-Ph |
| | (16) 3-Me-4-Cl-Ph | (16) 3-Me-4-Cl-Ph |
| | (17) 3-Cl-5-OBu-Ph | (17) 3-Cl-5-OBu-Ph |

## Table 3

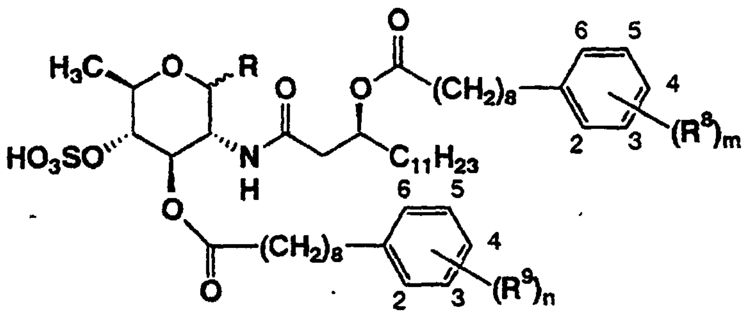

(I-3)

| R | 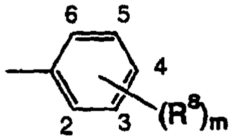 | 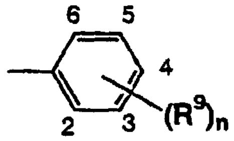 |
|---|---|---|
| (1) H<br>(2) OH<br>(3) OMe | (1) Ph<br>(2) 2-Cl-Ph<br>(3) 2-Me-Ph<br>(4) 2-OBu-Ph<br>(5) 3-Cl-Ph<br>(6) 3-Me-Ph<br>(7) 3-OBu-Ph<br>(8) 4-Cl-Ph<br>(9) 4-Me-Ph<br>(10) 4-OBu-Ph<br>(11) 2,4-diCl-Ph<br>(12) 3,4-diCl-Ph<br>(13) 2,4,6-triCl-Ph<br>(14) 2,4-diOBu-Ph<br>(15) 3,4-diOBu-Ph<br>(16) 3-Me-4-Cl-Ph<br>(17) 3-Cl-5-OBu-Ph | (1) Ph<br>(2) 2-Cl-Ph<br>(3) 2-Me-Ph<br>(4) 2-OBu-Ph<br>(5) 3-Cl-Ph<br>(6) 3-Me-Ph<br>(7) 3-OBu-Ph<br>(8) 4-Cl-Ph<br>(9) 4-Me-Ph<br>(10) 4-OBu-Ph<br>(11) 2,4-diCl-Ph<br>(12) 3,4-diCl-Ph<br>(13) 2,4,6-triCl-Ph<br>(14) 2,4-diOBu-Ph<br>(15) 3,4-diOBu-Ph<br>(16) 3-Me-4-Cl-Ph<br>(17) 3-Cl-5-OBu-Ph |

## Table 4

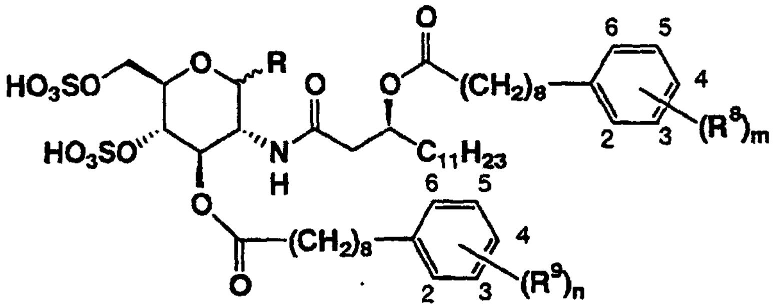

(I-4)

| R | 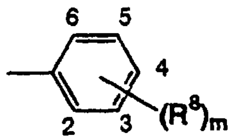 | 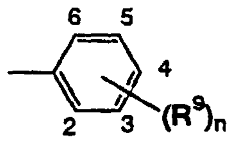 |
|---|---|---|
| (1) H | (1) Ph | (1) Ph |
| (2) OH | (2) 2-Cl-Ph | (2) 2-Cl-Ph |
| (3) OMe | (3) 2-Me-Ph | (3) 2-Me-Ph |
| | (4) 2-OBu-Ph | (4) 2-OBu-Ph |
| | (5) 3-Cl-Ph | (5) 3-Cl-Ph |
| | (6) 3-Me-Ph | (6) 3-Me-Ph |
| | (7) 3-OBu-Ph | (7) 3-OBu-Ph |
| | (8) 4-Cl-Ph | (8) 4-Cl-Ph |
| | (9) 4-Me-Ph | (9) 4-Me-Ph |
| | (10) 4-OBu-Ph | (10) 4-OBu-Ph |
| | (11) 2,4-diCl-Ph | (11) 2,4-diCl-Ph |
| | (12) 3,4-diCl-Ph | (12) 3,4-diCl-Ph |
| | (13) 2,4,6-triCl-Ph | (13) 2,4,6-triCl-Ph |
| | (14) 2,4-diOBu-Ph | (14) 2,4-diOBu-Ph |
| | (15) 3,4-diOBu-Ph | (15) 3,4-diOBu-Ph |
| | (16) 3-Me-4-Cl-Ph | (16) 3-Me-4-Cl-Ph |
| | (17) 3-Cl-5-OBu-Ph | (17) 3-Cl-5-OBu-Ph |

## Table 5

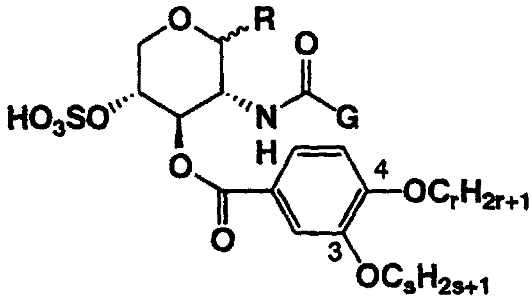

(I-5)

| R | G | 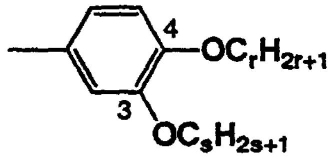 |
|---|---|---|
| (1) H | (1) 3,4-di$OC_6H_{13}$-Ph | (1) 3,4-di$OC_6H_{13}$-Ph |
| (2) OH | (2) 3,4-di$OC_7H_{15}$-Ph | (2) 3,4-di$OC_7H_{15}$-Ph |
| (3) OMe | (3) 3,4-di$OC_8H_{17}$-Ph | (3) 3,4-di$OC_8H_{17}$-Ph |
| | (4) 3,4-di$OC_9H_{19}$-Ph | (4) 3,4-di$OC_9H_{19}$-Ph |
| | (5) 3,4-di$OC_{10}H_{21}$-Ph | (5) 3,4-di$OC_{10}H_{21}$-Ph |
| | (6) 3,4-di$OC_{11}H_{23}$-Ph | (6) 3,4-di$OC_{11}H_{23}$-Ph |
| | (7) 3,4-di$OC_{12}H_{25}$-Ph | (7) 3,4-di$OC_{12}H_{25}$-Ph |
| | (8) $C_{13}H_{27}$ | |

## Table 6

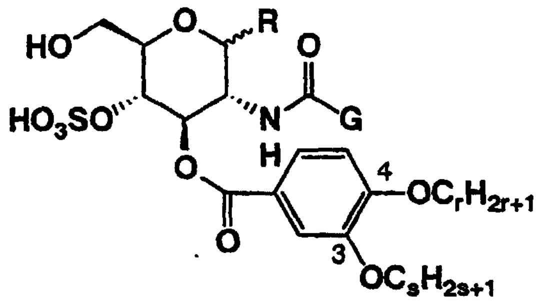

(I-6)

| R | G | 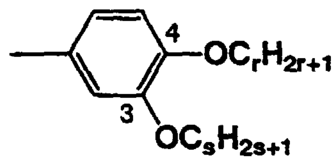 |
|---|---|---|
| (1) H | (1) 3,4-di$OC_6H_{13}$-Ph | (1) 3,4-di$OC_6H_{13}$-Ph |
| (2) OH | (2) 3,4-di$OC_7H_{15}$-Ph | (2) 3,4-di$OC_7H_{15}$-Ph |
| (3) OMe | (3) 3,4-di$OC_8H_{17}$-Ph | (3) 3,4-di$OC_8H_{17}$-Ph |
| | (4) 3,4-di$OC_9H_{19}$-Ph | (4) 3,4-di$OC_9H_{19}$-Ph |
| | (5) 3,4-di$OC_{10}H_{21}$-Ph | (5) 3,4-di$OC_{10}H_{21}$-Ph |
| | (6) 3,4-di$OC_{11}H_{23}$-Ph | (6) 3,4-di$OC_{11}H_{23}$-Ph |
| | (7) 3,4-di$OC_{12}H_{25}$-Ph | (7) 3,4-di$OC_{12}H_{25}$-Ph |
| | (8) $C_{13}H_{27}$ | |

**Table 7**

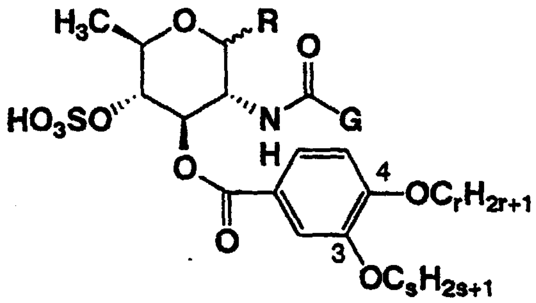

(I-7)

| R | G | 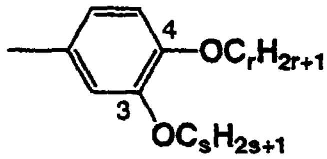 |
|---|---|---|
| (1) H | (1) 3,4-di$OC_6H_{13}$-Ph | (1) 3,4-di$OC_6H_{13}$-Ph |
| (2) OH | (2) 3,4-di$OC_7H_{15}$-Ph | (2) 3,4-di$OC_7H_{15}$-Ph |
| (3) OMe | (3) 3,4-di$OC_8H_{17}$-Ph | (3) 3,4-di$OC_8H_{17}$-Ph |
| | (4) 3,4-di$OC_9H_{19}$-Ph | (4) 3,4-di$OC_9H_{19}$-Ph |
| | (5) 3,4-di$OC_{10}H_{21}$-Ph | (5) 3,4-di$OC_{10}H_{21}$-Ph |
| | (6) 3,4-di$OC_{11}H_{23}$-Ph | (6) 3,4-di$OC_{11}H_{23}$-Ph |
| | (7) 3,4-di$OC_{12}H_{25}$-Ph | (7) 3,4-di$OC_{12}H_{25}$-Ph |
| | (8) $C_{13}H_{27}$ | |

## Table 8

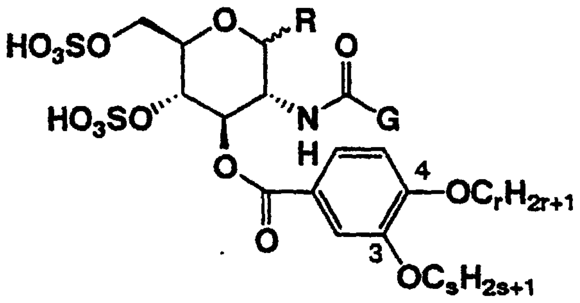

(I-8)

| R | G | 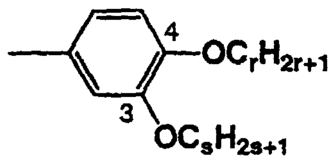 |
|---|---|---|
| (1) H | (1) 3,4-diOC$_6$H$_{13}$-Ph | (1) 3,4-diOC$_6$H$_{13}$-Ph |
| (2) OH | (2) 3,4-diOC$_7$H$_{15}$-Ph | (2) 3,4-diOC$_7$H$_{15}$-Ph |
| (3) OMe | (3) 3,4-diOC$_8$H$_{17}$-Ph | (3) 3,4-diOC$_8$H$_{17}$-Ph |
| | (4) 3,4-diOC$_9$H$_{19}$-Ph | (4) 3,4-diOC$_9$H$_{19}$-Ph |
| | (5) 3,4-diOC$_{10}$H$_{21}$-Ph | (5) 3,4-diOC$_{10}$H$_{21}$-Ph |
| | (6) 3,4-diOC$_{11}$H$_{23}$-Ph | (6) 3,4-diOC$_{11}$H$_{23}$-Ph |
| | (7) 3,4-diOC$_{12}$H$_{25}$-Ph | (7) 3,4-diOC$_{12}$H$_{25}$-Ph |
| | (8) C$_{13}$H$_{27}$ | |

**Table 9**

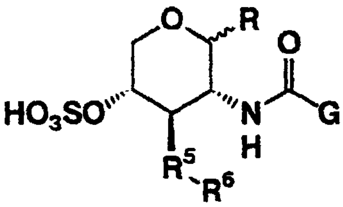

(I-9)

| R | —G | —$R^5$—$R^6$ |
|---|---|---|
| (1) H | (1) $-C_9H_{19}$ | (1) $-O-CO-C_5H_{11}$ |
| (2) OH | (2) $-C_{11}H_{23}$ | (2) $-O-CO-C_9H_{19}$ |
| (3) OMe | (3) $-C_{13}H_{27}$ | (3) $-O-CO-C_{11}H_{23}$ |
| | (4) $-C_{15}H_{31}$ | (4) $-O-CO-C_{13}H_{27}$ |
| | (5) $-C_{17}H_{35}$ | (5) $-O-CO-C_{15}H_{31}$ |
| | (6) 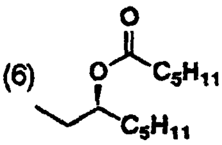 (7) 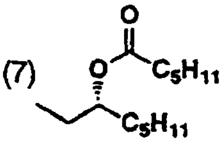 | (6) $-O-CO-C_{17}H_{35}$ |
| | (8) 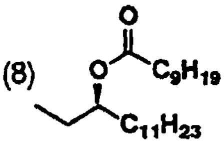 (9) 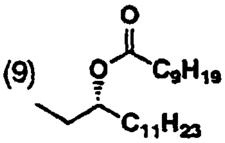 | (7) $-O-CO-C_{19}H_{39}$ |
| | (10) 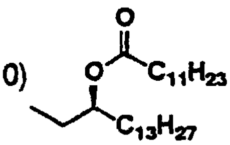 (11) 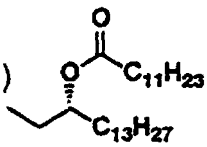 | |
| | (12) 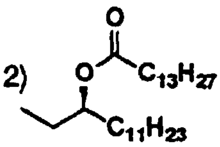 (13) 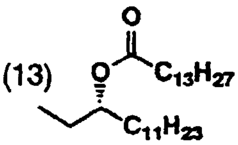 | |
| | (14) 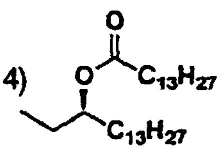 (15) 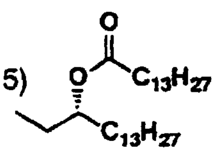 | |

## Table 10

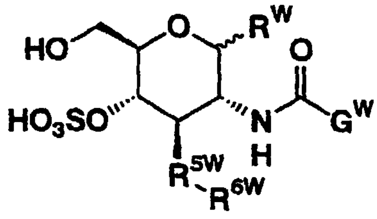

(W-10)

| $R^W$ | $-G^W$ | $-R^{5W}-R^{6W}$ |
|---|---|---|
| (1) H | (1) $-C_9H_{19}$ | (1) $-O-CO-C_5H_{11}$ |
| (2) OH | (2) $-C_{11}H_{23}$ | (2) $-O-CO-C_9H_{19}$ |
| (3) OMe | (3) $-C_{13}H_{27}$ | (3) $-O-CO-C_{11}H_{23}$ |
| | (4) $-C_{15}H_{31}$ | (4) $-O-CO-C_{13}H_{27}$ |
| | (5) $-C_{17}H_{35}$ | (5) $-O-CO-C_{15}H_{31}$ |
| | (6) 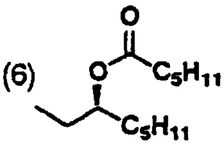 (7) 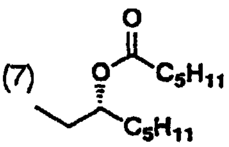 | (6) $-O-CO-C_{17}H_{35}$ |
| | (8) 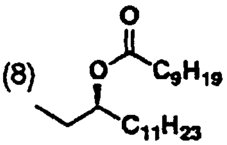 (9) 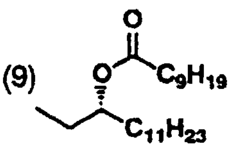 | (7) $-O-CO-C_{19}H_{39}$ |
| | (10) 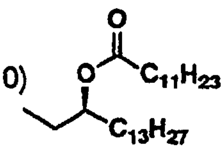 (11) 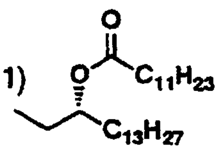 | |
| | (12) 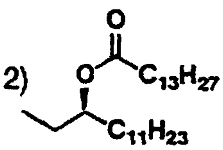 (13) 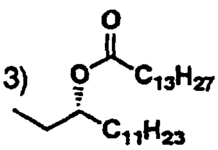 | |
| | (14) 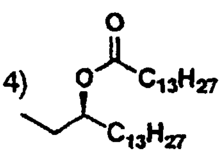 (15) 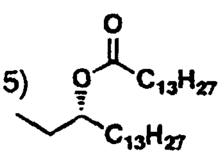 | |

## Table 11

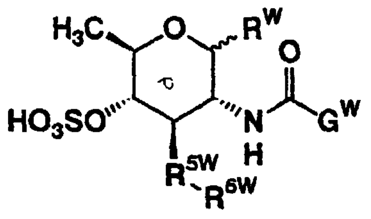

(W-11)

| $R^W$ | $-G^W$ | $-R^{5W}-R^{6W}$ |
|---|---|---|
| (1) H | (1) $-C_9H_{19}$ | (1) $-O-CO-C_5H_{11}$ |
| (2) OH | (2) $-C_{11}H_{23}$ | (2) $-O-CO-C_9H_{19}$ |
| (3) OMe | (3) $-C_{13}H_{27}$ | (3) $-O-CO-C_{11}H_{23}$ |
| | (4) $-C_{15}H_{31}$ | (4) $-O-CO-C_{13}H_{27}$ |
| | (5) $-C_{17}H_{35}$ | (5) $-O-CO-C_{15}H_{31}$ |
| | (6) 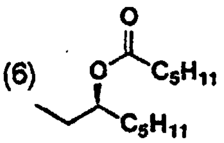 (7) 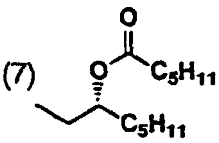 | (6) $-O-CO-C_{17}H_{35}$ |
| | (8) 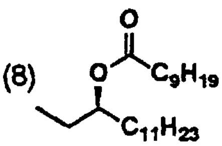 (9) 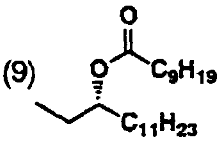 | (7) $-O-CO-C_{19}H_{39}$ |
| | (10) 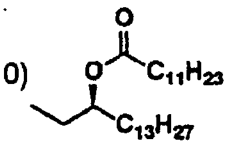 (11) 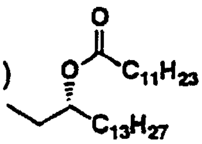 | |
| | (12) 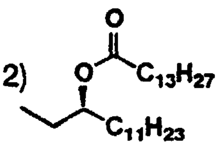 (13) 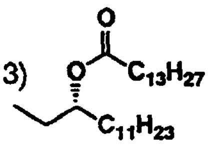 | |
| | (14) 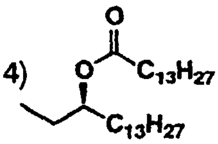 (15) 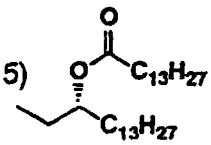 | |

## Table 12

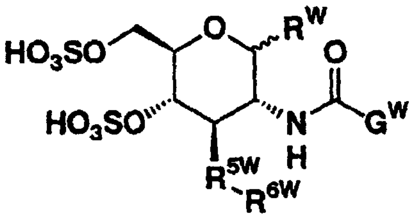

(W-12)

| $R^W$ | $—G^W$ | $—R^{5W}—R^{6W}$ |
|---|---|---|
| (1) H | (1) $-C_9H_{19}$ | (1) $-O-CO-C_5H_{11}$ |
| (2) OH | (2) $-C_{11}H_{23}$ | (2) $-O-CO-C_9H_{19}$ |
| (3) OMe | (3) $-C_{13}H_{27}$ | (3) $-O-CO-C_{11}H_{23}$ |
| | (4) $-C_{15}H_{31}$ | (4) $-O-CO-C_{13}H_{27}$ |
| | (5) $-C_{17}H_{35}$ | (5) $-O-CO-C_{15}H_{31}$ |
| | | (6) $-O-CO-C_{17}H_{35}$ |
| | (6) 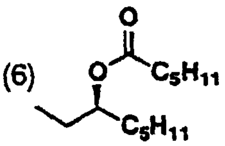 (7) 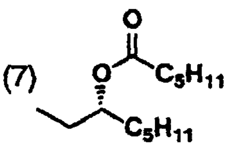 | (7) $-O-CO-C_{19}H_{39}$ |
| | (8) 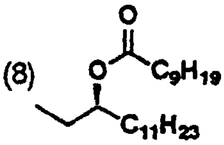 (9) 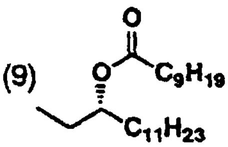 | |
| | (10) 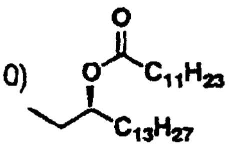 (11) 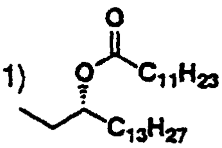 | |
| | (12) 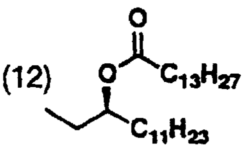 (13) 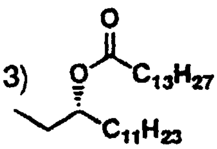 | |
| | (14) 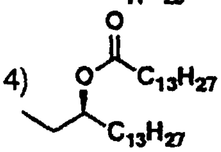 (15) 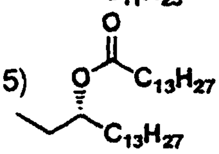 | |

## Table 13

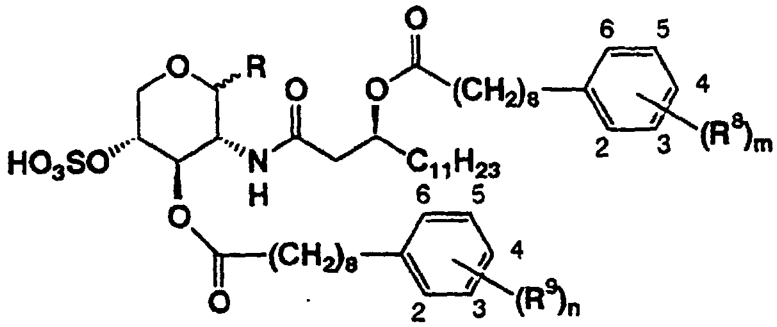

(I-1)

| No. | R | 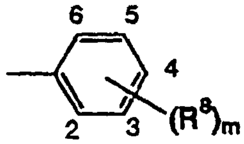 | 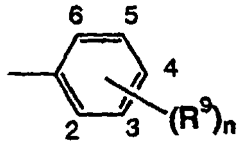 |
|---|---|---|---|
| 1 | H | $-C_6H_5$ | $-C_6H_5$ |
| 2 | H | $-C_6H_4$-(4-OMe) | $-C_6H_4$-(4-OMe) |
| 3 | H | $-C_6H_3$-(3,4-diOMe) | $-C_6H_3$-(3,4-diOMe) |
| 4 | H | $-C_6H_4$-(4-Me) | $-C_6H_4$-(4-Me) |
| 5 | H | $-C_6H_3$-(3,4-diMe) | $-C_6H_3$-(3,4-diMe) |
| 6 | H | $-C_6H_4$-(4-Cl) | $-C_6H_4$-(4-Cl) |
| 7 | H | $-C_6H_3$-(3,4-diCl) | $-C_6H_3$-(3,4-diCl) |
| 8 | OH | $-C_6H_5$ | $-C_6H_5$ |
| 9 | OH | $-C_6H_4$-(4-OMe) | $-C_6H_4$-(4-OMe) |
| 10 | OH | $-C_6H_3$-(3,4-diOMe) | $-C_6H_3$-(3,4-diOMe) |
| 11 | OH | $-C_6H_4$-(4-Me) | $-C_6H_4$-(4-Me) |
| 12 | OH | $-C_6H_3$-(3,4-diMe) | $-C_6H_3$-(3,4-diMe) |
| 13 | OH | $-C_6H_4$-(4-Cl) | $-C_6H_4$-(4-Cl) |
| 14 | OH | $-C_6H_3$-(3,4-diCl) | $-C_6H_3$-(3,4-diCl) |
| 15 | OMe | $-C_6H_5$ | $-C_6H_5$ |
| 16 | OMe | $-C_6H_4$-(4-OMe) | $-C_6H_4$-(4-OMe) |
| 17 | OMe | $-C_6H_3$-(3,4-diOMe) | $-C_6H_3$-(3,4-diOMe) |
| 18 | OMe | $-C_6H_4$-(4-Me) | $-C_6H_4$-(4-Me) |
| 19 | OMe | $-C_6H_3$-(3,4-diMe) | $-C_6H_3$-(3,4-diMe) |
| 20 | OMe | $-C_6H_4$-(4-Cl) | $-C_6H_4$-(4-Cl) |
| 21 | OMe | $-C_6H_3$-(3,4-diCl) | $-C_6H_3$-(3,4-diCl) |

## Table 14

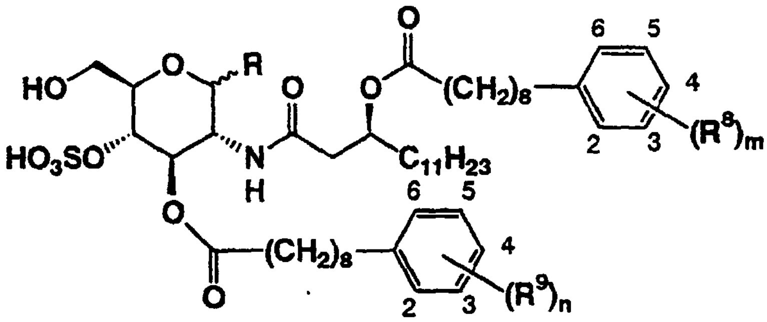

(I-2)

| No. | R | 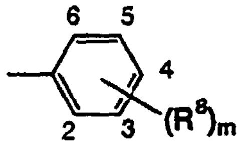 | 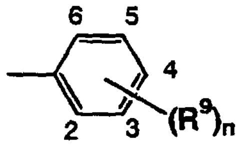 |
|---|---|---|---|
| 1 | H | $-C_6H_5$ | $-C_6H_5$ |
| 2 | H | $-C_6H_4$-(4-OMe) | $-C_6H_4$-(4-OMe) |
| 3 | H | $-C_6H_3$-(3,4-diOMe) | $-C_6H_3$-(3,4-diOMe) |
| 4 | H | $-C_6H_4$-(4-Me) | $-C_6H_4$-(4-Me) |
| 5 | H | $-C_6H_3$-(3,4-diMe) | $-C_6H_3$-(3,4-diMe) |
| 6 | H | $-C_6H_4$-(4-Cl) | $-C_6H_4$-(4-Cl) |
| 7 | H | $-C_6H_3$-(3,4-diCl) | $-C_6H_3$-(3,4-diCl) |
| 8 | OH | $-C_6H_5$ | $-C_6H_5$ |
| 9 | OH | $-C_6H_4$-(4-OMe) | $-C_6H_4$-(4-OMe) |
| 10 | OH | $-C_6H_3$-(3,4-diOMe) | $-C_6H_3$-(3,4-diOMe) |
| 11 | OH | $-C_6H_4$-(4-Me) | $-C_6H_4$-(4-Me) |
| 12 | OH | $-C_6H_3$-(3,4-diMe) | $-C_6H_3$-(3,4-diMe) |
| 13 | OH | $-C_6H_4$-(4-Cl) | $-C_6H_4$-(4-Cl) |
| 14 | OH | $-C_6H_3$-(3,4-diCl) | $-C_6H_3$-(3,4-diCl) |
| 15 | OMe | $-C_6H_5$ | $-C_6H_5$ |
| 16 | OMe | $-C_6H_4$-(4-OMe) | $-C_6H_4$-(4-OMe) |
| 17 | OMe | $-C_6H_3$-(3,4-diOMe) | $-C_6H_3$-(3,4-diOMe) |
| 18 | OMe | $-C_6H_4$-(4-Me) | $-C_6H_4$-(4-Me) |
| 19 | OMe | $-C_6H_3$-(3,4-diMe) | $-C_6H_3$-(3,4-diMe) |
| 20 | OMe | $-C_6H_4$-(4-Cl) | $-C_6H_4$-(4-Cl) |
| 21 | OMe | $-C_6H_3$-(3,4-diCl) | $-C_6H_3$-(3,4-diCl) |

**Table 15**

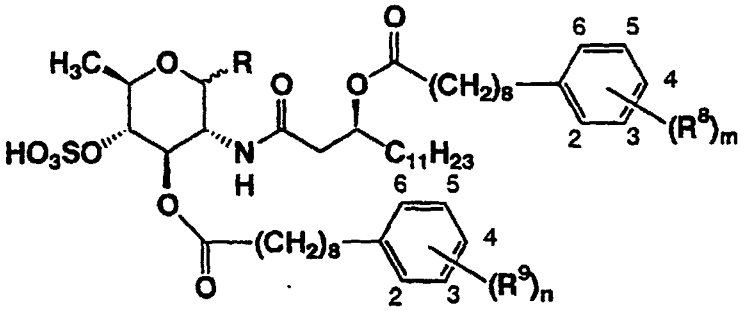

(I-3)

| No. | R | 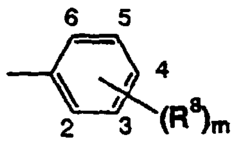 | 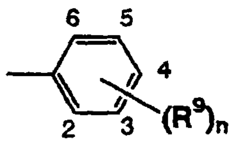 |
|---|---|---|---|
| 1 | H | $-C_6H_5$ | $-C_6H_5$ |
| 2 | H | $-C_6H_4$-(4-OMe) | $-C_6H_4$-(4-OMe) |
| 3 | H | $-C_6H_3$-(3,4-diOMe) | $-C_6H_3$-(3,4-diOMe) |
| 4 | H | $-C_6H_4$-(4-Me) | $-C_6H_4$-(4-Me) |
| 5 | H | $-C_6H_3$-(3,4-diMe) | $-C_6H_3$-(3,4-diMe) |
| 6 | H | $-C_6H_4$-(4-Cl) | $-C_6H_4$-(4-Cl) |
| 7 | H | $-C_6H_3$-(3,4-diCl) | $-C_6H_3$-(3,4-diCl) |
| 8 | OH | $-C_6H_5$ | $-C_6H_5$ |
| 9 | OH | $-C_6H_4$-(4-OMe) | $-C_6H_4$-(4-OMe) |
| 10 | OH | $-C_6H_3$-(3,4-diOMe) | $-C_6H_3$-(3,4-diOMe) |
| 11 | OH | $-C_6H_4$-(4-Me) | $-C_6H_4$-(4-Me) |
| 12 | OH | $-C_6H_3$-(3,4-diMe) | $-C_6H_3$-(3,4-diMe) |
| 13 | OH | $-C_6H_4$-(4-Cl) | $-C_6H_4$-(4-Cl) |
| 14 | OH | $-C_6H_3$-(3,4-diCl) | $-C_6H_3$-(3,4-diCl) |
| 15 | OMe | $-C_6H_5$ | $-C_6H_5$ |
| 16 | OMe | $-C_6H_4$-(4-OMe) | $-C_6H_4$-(4-OMe) |
| 17 | OMe | $-C_6H_3$-(3,4-diOMe) | $-C_6H_3$-(3,4-diOMe) |
| 18 | OMe | $-C_6H_4$-(4-Me) | $-C_6H_4$-(4-Me) |
| 19 | OMe | $-C_6H_3$-(3,4-diMe) | $-C_6H_3$-(3,4-diMe) |
| 20 | OMe | $-C_6H_4$-(4-Cl) | $-C_6H_4$-(4-Cl) |
| 21 | OMe | $-C_6H_3$-(3,4-diCl) | $-C_6H_3$-(3,4-diCl) |

## Table 16

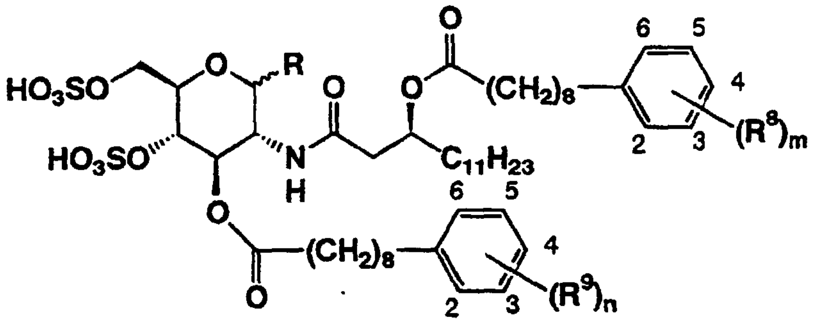

(I-4)

| No. | R | 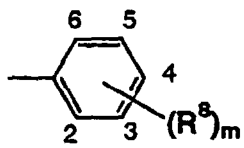 | 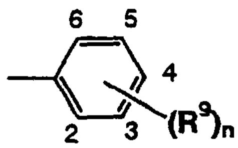 |
|---|---|---|---|
| 1 | H | $-C_6H_5$ | $-C_6H_5$ |
| 2 | H | $-C_6H_4$-(4-OMe) | $-C_6H_4$-(4-OMe) |
| 3 | H | $-C_6H_3$-(3,4-diOMe) | $-C_6H_3$-(3,4-diOMe) |
| 4 | H | $-C_6H_4$-(4-Me) | $-C_6H_4$-(4-Me) |
| 5 | H | $-C_6H_3$-(3,4-diMe) | $-C_6H_3$-(3,4-diMe) |
| 6 | H | $-C_6H_4$-(4-Cl) | $-C_6H_4$-(4-Cl) |
| 7 | H | $-C_6H_3$-(3,4-diCl) | $-C_6H_3$-(3,4-diCl) |
| 8 | OH | $-C_6H_5$ | $-C_6H_5$ |
| 9 | OH | $-C_6H_4$-(4-OMe) | $-C_6H_4$-(4-OMe) |
| 10 | OH | $-C_6H_3$-(3,4-diOMe) | $-C_6H_3$-(3,4-diOMe) |
| 11 | OH | $-C_6H_4$-(4-Me) | $-C_6H_4$-(4-Me) |
| 12 | OH | $-C_6H_3$-(3,4-diMe) | $-C_6H_3$-(3,4-diMe) |
| 13 | OH | $-C_6H_4$-(4-Cl) | $-C_6H_4$-(4-Cl) |
| 14 | OH | $-C_6H_3$-(3,4-diCl) | $-C_6H_3$-(3,4-diCl) |
| 15 | OMe | $-C_6H_5$ | $-C_6H_5$ |
| 16 | OMe | $-C_6H_4$-(4-OMe) | $-C_6H_4$-(4-OMe) |
| 17 | OMe | $-C_6H_3$-(3,4-diOMe) | $-C_6H_3$-(3,4-diOMe) |
| 18 | OMe | $-C_6H_4$-(4-Me) | $-C_6H_4$-(4-Me) |
| 19 | OMe | $-C_6H_3$-(3,4-diMe) | $-C_6H_3$-(3,4-diMe) |
| 20 | OMe | $-C_6H_4$-(4-Cl) | $-C_6H_4$-(4-Cl) |
| 21 | OMe | $-C_6H_3$-(3,4-diCl) | $-C_6H_3$-(3,4-diCl) |

## Table 17

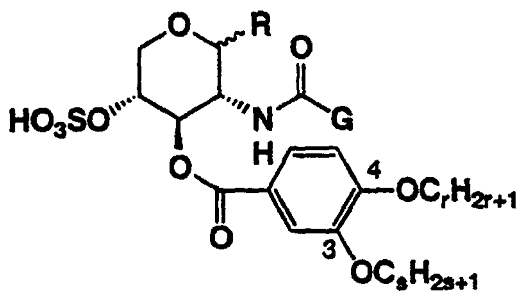

(I-5)

| No. | R | —G | 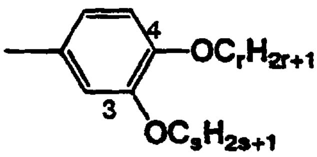 |
|---|---|---|---|
| 1 | H | $-C_{13}H_{27}$ | $-C_6H_3-(3,4-diOC_6H_{13})$ |
| 2 | H | $-C_{13}H_{27}$ | $-C_6H_3-(3,4-diOC_8H_{17})$ |
| 3 | H | $-C_{13}H_{27}$ | $-C_6H_3-(3,4-diOC_{10}H_{21})$ |
| 4 | H | $-C_{13}H_{27}$ | $-C_6H_3-(3,4-diOC_{12}H_{25})$ |
| 5 | H | $-C_6H_3-(3,4-diOC_6H_{13})$ | $-C_6H_3-(3,4-diOC_6H_{13})$ |
| 6 | H | $-C_6H_3-(3,4-diOC_8H_{17})$ | $-C_6H_3-(3,4-diOC_8H_{17})$ |
| 7 | H | $-C_6H_3-(3,4-diOC_{10}H_{21})$ | $-C_6H_3-(3,4-diOC_{10}H_{21})$ |
| 8 | OH | $-C_{13}H_{27}$ | $-C_6H_3-(3,4-diOC_6H_{13})$ |
| 9 | OH | $-C_{13}H_{27}$ | $-C_6H_3-(3,4-diOC_8H_{17})$ |
| 10 | OH | $-C_{13}H_{27}$ | $-C_6H_3-(3,4-diOC_{10}H_{21})$ |
| 11 | OH | $-C_{13}H_{27}$ | $-C_6H_3-(3,4-diOC_{12}H_{25})$ |
| 12 | OH | $-C_6H_3-(3,4-diOC_6H_{13})$ | $-C_6H_3-(3,4-diOC_6H_{13})$ |
| 13 | OH | $-C_6H_3-(3,4-diOC_8H_{17})$ | $-C_6H_3-(3,4-diOC_8H_{17})$ |
| 14 | OH | $-C_6H_3-(3,4-diOC_{10}H_{21})$ | $-C_6H_3-(3,4-diOC_{10}H_{21})$ |
| 15 | OMe | $-C_{13}H_{27}$ | $-C_6H_3-(3,4-diOC_6H_{13})$ |
| 16 | OMe | $-C_{13}H_{27}$ | $-C_6H_3-(3,4-diOC_8H_{17})$ |
| 17 | OMe | $-C_{13}H_{27}$ | $-C_6H_3-(3,4-diOC_{10}H_{21})$ |
| 18 | OMe | $-C_{13}H_{27}$ | $-C_6H_3-(3,4-diOC_{12}H_{25})$ |
| 19 | OMe | $-C_6H_3-(3,4-diOC_6H_{13})$ | $-C_6H_3-(3,4-diOC_6H_{13})$ |
| 20 | OMe | $-C_6H_3-(3,4-diOC_8H_{17})$ | $-C_6H_3-(3,4-diOC_8H_{17})$ |
| 21 | OMe | $-C_6H_3-(3,4-diOC_{10}H_{21})$ | $-C_6H_3-(3,4-diOC_{10}H_{21})$ |

## Table 18

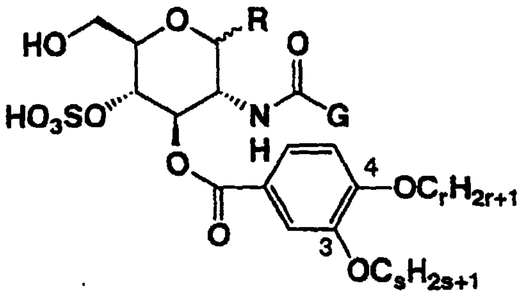

(I-6)

| No. | R | —G | 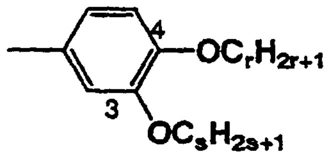 |
|---|---|---|---|
| 1 | H | $-C_{13}H_{27}$ | $-C_6H_3$-(3,4-di$OC_6H_{13}$) |
| 2 | H | $-C_{13}H_{27}$ | $-C_6H_3$-(3,4-di$OC_8H_{17}$) |
| 3 | H | $-C_{13}H_{27}$ | $-C_6H_3$-(3,4-di$OC_{10}H_{21}$) |
| 4 | H | $-C_{13}H_{27}$ | $-C_6H_3$-(3,4-di$OC_{12}H_{25}$) |
| 5 | H | $-C_6H_3$-(3,4-di$OC_6H_{13}$) | $-C_6H_3$-(3,4-di$OC_6H_{13}$) |
| 6 | H | $-C_6H_3$-(3,4-di$OC_8H_{17}$) | $-C_6H_3$-(3,4-di$OC_8H_{17}$) |
| 7 | H | $-C_6H_3$-(3,4-di$OC_{10}H_{21}$) | $-C_6H_3$-(3,4-di$OC_{10}H_{21}$) |
| 8 | OH | $-C_{13}H_{27}$ | $-C_6H_3$-(3,4-di$OC_6H_{13}$) |
| 9 | OH | $-C_{13}H_{27}$ | $-C_6H_3$-(3,4-di$OC_8H_{17}$) |
| 10 | OH | $-C_{13}H_{27}$ | $-C_6H_3$-(3,4-di$OC_{10}H_{21}$) |
| 11 | OH | $-C_{13}H_{27}$ | $-C_6H_3$-(3,4-di$OC_{12}H_{25}$) |
| 12 | OH | $-C_6H_3$-(3,4-di$OC_6H_{13}$) | $-C_6H_3$-(3,4-di$OC_6H_{13}$) |
| 13 | OH | $-C_6H_3$-(3,4-di$OC_8H_{17}$) | $-C_6H_3$-(3,4-di$OC_8H_{17}$) |
| 14 | OH | $-C_6H_3$-(3,4-di$OC_{10}H_{21}$) | $-C_6H_3$-(3,4-di$OC_{10}H_{21}$) |
| 15 | OMe | $-C_{13}H_{27}$ | $-C_6H_3$-(3,4-di$OC_6H_{13}$) |
| 16 | OMe | $-C_{13}H_{27}$ | $-C_6H_3$-(3,4-di$OC_8H_{17}$) |
| 17 | OMe | $-C_{13}H_{27}$ | $-C_6H_3$-(3,4-di$OC_{10}H_{21}$) |
| 18 | OMe | $-C_{13}H_{27}$ | $-C_6H_3$-(3,4-di$OC_{12}H_{25}$) |
| 19 | OMe | $-C_6H_3$-(3,4-di$OC_6H_{13}$) | $-C_6H_3$-(3,4-di$OC_6H_{13}$) |
| 20 | OMe | $-C_6H_3$-(3,4-di$OC_8H_{17}$) | $-C_6H_3$-(3,4-di$OC_8H_{17}$) |
| 21 | OMe | $-C_6H_3$-(3,4-di$OC_{10}H_{21}$) | $-C_6H_3$-(3,4-di$OC_{10}H_{21}$) |

## Table 19

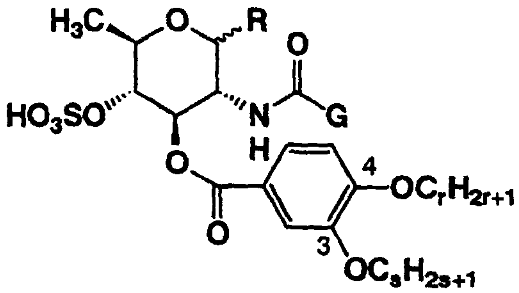

(I-7)

| No. | R | —G | 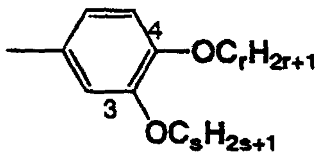 |
|---|---|---|---|
| 1 | H | $-C_{13}H_{27}$ | $-C_6H_3-(3,4\text{-di}OC_6H_{13})$ |
| 2 | H | $-C_{13}H_{27}$ | $-C_6H_3-(3,4\text{-di}OC_8H_{17})$ |
| 3 | H | $-C_{13}H_{27}$ | $-C_6H_3-(3,4\text{-di}OC_{10}H_{21})$ |
| 4 | H | $-C_{13}H_{27}$ | $-C_6H_3-(3,4\text{-di}OC_{12}H_{25})$ |
| 5 | H | $-C_6H_3-(3,4\text{-di}OC_6H_{13})$ | $-C_6H_3-(3,4\text{-di}OC_6H_{13})$ |
| 6 | H | $-C_6H_3-(3,4\text{-di}OC_8H_{17})$ | $-C_6H_3-(3,4\text{-di}OC_8H_{17})$ |
| 7 | H | $-C_6H_3-(3,4\text{-di}OC_{10}H_{21})$ | $-C_6H_3-(3,4\text{-di}OC_{10}H_{21})$ |
| 8 | OH | $-C_{13}H_{27}$ | $-C_6H_3-(3,4\text{-di}OC_6H_{13})$ |
| 9 | OH | $-C_{13}H_{27}$ | $-C_6H_3-(3,4\text{-di}OC_8H_{17})$ |
| 10 | OH | $-C_{13}H_{27}$ | $-C_6H_3-(3,4\text{-di}OC_{10}H_{21})$ |
| 11 | OH | $-C_{13}H_{27}$ | $-C_6H_3-(3,4\text{-di}OC_{12}H_{25})$ |
| 12 | OH | $-C_6H_3-(3,4\text{-di}OC_6H_{13})$ | $-C_6H_3-(3,4\text{-di}OC_6H_{13})$ |
| 13 | OH | $-C_6H_3-(3,4\text{-di}OC_8H_{17})$ | $-C_6H_3-(3,4\text{-di}OC_8H_{17})$ |
| 14 | OH | $-C_6H_3-(3,4\text{-di}OC_{10}H_{21})$ | $-C_6H_3-(3,4\text{-di}OC_{10}H_{21})$ |
| 15 | OMe | $-C_{13}H_{27}$ | $-C_6H_3-(3,4\text{-di}OC_6H_{13})$ |
| 16 | OMe | $-C_{13}H_{27}$ | $-C_6H_3-(3,4\text{-di}OC_8H_{17})$ |
| 17 | OMe | $-C_{13}H_{27}$ | $-C_6H_3-(3,4\text{-di}OC_{10}H_{21})$ |
| 18 | OMe | $-C_{13}H_{27}$ | $-C_6H_3-(3,4\text{-di}OC_{12}H_{25})$ |
| 19 | OMe | $-C_6H_3-(3,4\text{-di}OC_6H_{13})$ | $-C_6H_3-(3,4\text{-di}OC_6H_{13})$ |
| 20 | OMe | $-C_6H_3-(3,4\text{-di}OC_8H_{17})$ | $-C_6H_3-(3,4\text{-di}OC_8H_{17})$ |
| 21 | OMe | $-C_6H_3-(3,4\text{-di}OC_{10}H_{21})$ | $-C_6H_3-(3,4\text{-di}OC_{10}H_{21})$ |

## Table 20

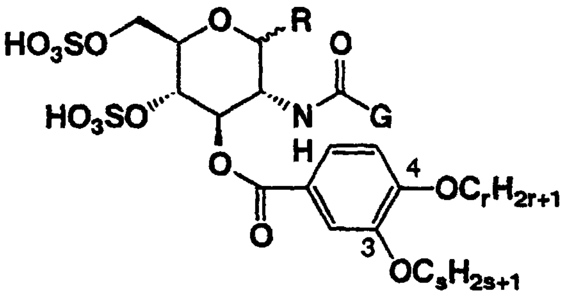

(I-8)

| No. | R | —G | 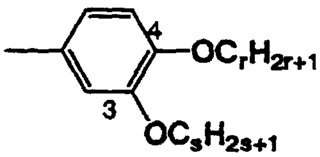 |
|---|---|---|---|
| 1 | H | $-C_{13}H_{27}$ | $-C_6H_3-(3,4-diOC_6H_{13})$ |
| 2 | H | $-C_{13}H_{27}$ | $-C_6H_3-(3,4-diOC_8H_{17})$ |
| 3 | H | $-C_{13}H_{27}$ | $-C_6H_3-(3,4-diOC_{10}H_{21})$ |
| 4 | H | $-C_{13}H_{27}$ | $-C_6H_3-(3,4-diOC_{12}H_{25})$ |
| 5 | H | $-C_6H_3-(3,4-diOC_6H_{13})$ | $-C_6H_3-(3,4-diOC_6H_{13})$ |
| 6 | H | $-C_6H_3-(3,4-diOC_8H_{17})$ | $-C_6H_3-(3,4-diOC_8H_{17})$ |
| 7 | H | $-C_6H_3-(3,4-diOC_{10}H_{21})$ | $-C_6H_3-(3,4-diOC_{10}H_{21})$ |
| 8 | OH | $-C_{13}H_{27}$ | $-C_6H_3-(3,4-diOC_6H_{13})$ |
| 9 | OH | $-C_{13}H_{27}$ | $-C_6H_3-(3,4-diOC_8H_{17})$ |
| 10 | OH | $-C_{13}H_{27}$ | $-C_6H_3-(3,4-diOC_{10}H_{21})$ |
| 11 | OH | $-C_{13}H_{27}$ | $-C_6H_3-(3,4-diOC_{12}H_{25})$ |
| 12 | OH | $-C_6H_3-(3,4-diOC_6H_{13})$ | $-C_6H_3-(3,4-diOC_6H_{13})$ |
| 13 | OH | $-C_6H_3-(3,4-diOC_8H_{17})$ | $-C_6H_3-(3,4-diOC_8H_{17})$ |
| 14 | OH | $-C_6H_3-(3,4-diOC_{10}H_{21})$ | $-C_6H_3-(3,4-diOC_{10}H_{21})$ |
| 15 | OMe | $-C_{13}H_{27}$ | $-C_6H_3-(3,4-diOC_6H_{13})$ |
| 16 | OMe | $-C_{13}H_{27}$ | $-C_6H_3-(3,4-diOC_8H_{17})$ |
| 17 | OMe | $-C_{13}H_{27}$ | $-C_6H_3-(3,4-diOC_{10}H_{21})$ |
| 18 | OMe | $-C_{13}H_{27}$ | $-C_6H_3-(3,4-diOC_{12}H_{25})$ |
| 19 | OMe | $-C_6H_3-(3,4-diOC_6H_{13})$ | $-C_6H_3-(3,4-diOC_6H_{13})$ |
| 20 | OMe | $-C_6H_3-(3,4-diOC_8H_{17})$ | $-C_6H_3-(3,4-diOC_8H_{17})$ |
| 21 | OMe | $-C_6H_3-(3,4-diOC_{10}H_{21})$ | $-C_6H_3-(3,4-diOC_{10}H_{21})$ |

## Table 21

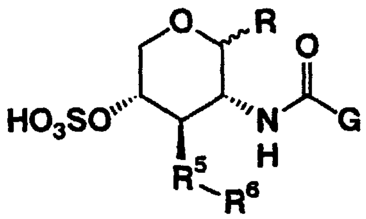

(I-9)

| No. | R | —G | —$R^5$—$R^6$ |
|---|---|---|---|
| 1 | H | $-C_{13}H_{27}$ | $-C_{13}H_{27}$ |
| 2 | H | (R)-$CH_2CH(OCOC_5H_{11})C_{11}H_{23}$ | $-C_5H_{11}$ |
| 3 | H | (S)-$CH_2CH(OCOC_5H_{11})C_{11}H_{23}$ | $-C_5H_{11}$ |
| 4 | H | (R)-$CH_2CH(OCOC_9H_{19})C_{11}H_{23}$ | $-C_9H_{19}$ |
| 5 | H | (S)-$CH_2CH(OCOC_9H_{19})C_{11}H_{23}$ | $-C_9H_{19}$ |
| 6 | H | (R)-$CH_2CH(OCOC_{13}H_{27})C_{13}H_{27}$ | $-C_{13}H_{27}$ |
| 7 | H | (S)-$CH_2CH(OCOC_{13}H_{27})C_{13}H_{27}$ | $-C_{13}H_{27}$ |
| 8 | OH | $-C_{13}H_{27}$ | $-C_{13}H_{27}$ |
| 9 | OH | (R)-$CH_2CH(OCOC_5H_{11})C_{11}H_{23}$ | $-C_5H_{11}$ |
| 10 | OH | (S)-$CH_2CH(OCOC_5H_{11})C_{11}H_{23}$ | $-C_5H_{11}$ |
| 11 | OH | (R)-$CH_2CH(OCOC_9H_{19})C_{11}H_{23}$ | $-C_9H_{19}$ |
| 12 | OH | (S)-$CH_2CH(OCOC_9H_{19})C_{11}H_{23}$ | $-C_9H_{19}$ |
| 13 | OH | (R)-$CH_2CH(OCOC_{13}H_{27})C_{13}H_{27}$( | $-C_{13}H_{27}$ |
| 14 | OH | S)-$CH_2CH(OCOC_{13}H_{27})C_{13}H_{27}$ | $-C_{13}H_{27}$ |
| 15 | OMe | $-C_{13}H_{27}$ | $-C_{13}H_{27}$ |
| 16 | OMe | (R)-$CH_2CH(OCOC_5H_{11})C_{11}H_{23}$ | $-C_5H_{11}$ |
| 17 | OMe | (S)-$CH_2CH(OCOC_5H_{11})C_{11}H_{23}$ | $-C_5H_{11}$ |
| 18 | OMe | (R)-$CH_2CH(OCOC_9H_{19})C_{11}H_{23}$ | $-C_9H_{19}$ |
| 19 | OMe | (S)-$CH_2CH(OCOC_9H_{19})C_{11}H_{23}$ | $-C_9H_{19}$ |
| 20 | OMe | (R)-$CH_2CH(OCOC_{13}H_{27})C_{13}H_{27}$( | $-C_{13}H_{27}$- |
| 21 | OMe | S)-$CH_2CH(OCOC_{13}H_{27})C_{13}H_{27}$ | $C_{13}H_{27}$ |

## Table 22

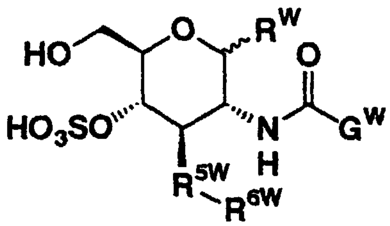

(W-10)

| No. | $R^W$ | $-G^W$ | $-R^{5W}-R^{6W}$ |
|---|---|---|---|
| 1 | H | $-C_{13}H_{27}$ | $-C_{13}H_{27}$ |
| 2 | H | (R)-$CH_2CH(OCOC_5H_{11})C_{11}H_{23}$ | $-C_5H_{11}$ |
| 3 | H | (S)-$CH_2CH(OCOC_5H_{11})C_{11}H_{23}$ | $-C_5H_{11}$ |
| 4 | H | (R)-$CH_2CH(OCOC_9H_{19})C_{11}H_{23}$ | $-C_9H_{19}$ |
| 5 | H | (S)-$CH_2CH(OCOC_9H_{19})C_{11}H_{23}$ | $-C_9H_{19}$ |
| 6 | H | (R)-$CH_2CH(OCOC_{13}H_{27})C_{13}H_{27}$ | $-C_{13}H_{27}$ |
| 7 | H | (S)-$CH_2CH(OCOC_{13}H_{27})C_{13}H_{27}$ | $-C_{13}H_{27}$ |
| 8 | OH | $-C_{13}H_{27}$ | $-C_{13}H_{27}$ |
| 9 | OH | (R)-$CH_2CH(OCOC_5H_{11})C_{11}H_{23}$ | $-C_5H_{11}$ |
| 10 | OH | (S)-$CH_2CH(OCOC_5H_{11})C_{11}H_{23}$ | $-C_5H_{11}$ |
| 11 | OH | (R)-$CH_2CH(OCOC_9H_{19})C_{11}H_{23}$ | $-C_9H_{19}$ |
| 12 | OH | (S)-$CH_2CH(OCOC_9H_{19})C_{11}H_{23}$ | $-C_9H_{19}$ |
| 13 | OH | (R)-$CH_2CH(OCOC_{13}H_{27})C_{13}H_{27}$( | $-C_{13}H_{27}$ |
| 14 | OH | S)-$CH_2CH(OCOC_{13}H_{27})C_{13}H_{27}$ | $-C_{13}H_{27}$ |
| 15 | OMe | $-C_{13}H_{27}$ | $-C_{13}H_{27}$ |
| 16 | OMe | (R)-$CH_2CH(OCOC_5H_{11})C_{11}H_{23}$ | $-C_5H_{11}$ |
| 17 | OMe | (S)-$CH_2CH(OCOC_5H_{11})C_{11}H_{23}$ | $-C_5H_{11}$ |
| 18 | OMe | (R)-$CH_2CH(OCOC_9H_{19})C_{11}H_{23}$ | $-C_9H_{19}$ |
| 19 | OMe | (S)-$CH_2CH(OCOC_9H_{19})C_{11}H_{23}$ | $-C_9H_{19}$ |
| 20 | OMe | (R)-$CH_2CH(OCOC_{13}H_{27})C_{13}H_{27}$( | $-C_{13}H_{27}$- |
| 21 | OMe | S)-$CH_2CH(OCOC_{13}H_{27})C_{13}H_{27}$ | $C_{13}H_{27}$ |

## Table 23

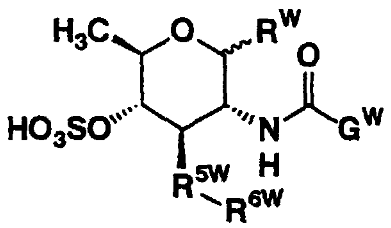

(W-11)

| No. | $R^W$ | $—G^W$ | $—R^{5W}—R^{6W}$ |
|---|---|---|---|
| 1 | H | $-C_{13}H_{27}$ | $-C_{13}H_{27}$ |
| 2 | H | (R)-$CH_2CH(OCOC_5H_{11})C_{11}H_{23}$ | $-C_5H_{11}$ |
| 3 | H | (S)-$CH_2CH(OCOC_5H_{11})C_{11}H_{23}$ | $-C_5H_{11}$ |
| 4 | H | (R)-$CH_2CH(OCOC_9H_{19})C_{11}H_{23}$ | $-C_9H_{19}$ |
| 5 | H | (S)-$CH_2CH(OCOC_9H_{19})C_{11}H_{23}$ | $-C_9H_{19}$ |
| 6 | H | (R)-$CH_2CH(OCOC_{13}H_{27})C_{13}H_{27}$ | $-C_{13}H_{27}$ |
| 7 | H | (S)-$CH_2CH(OCOC_{13}H_{27})C_{13}H_{27}$ | $-C_{13}H_{27}$ |
| 8 | OH | $-C_{13}H_{27}$ | $-C_{13}H_{27}$ |
| 9 | OH | (R)-$CH_2CH(OCOC_5H_{11})C_{11}H_{23}$ | $-C_5H_{11}$ |
| 10 | OH | (S)-$CH_2CH(OCOC_5H_{11})C_{11}H_{23}$ | $-C_5H_{11}$ |
| 11 | OH | (R)-$CH_2CH(OCOC_9H_{19})C_{11}H_{23}$ | $-C_9H_{19}$ |
| 12 | OH | (S)-$CH_2CH(OCOC_9H_{19})C_{11}H_{23}$ | $-C_9H_{19}$ |
| 13 | OH | (R)-$CH_2CH(OCOC_{13}H_{27})C_{13}H_{27}$( | $-C_{13}H_{27}$ |
| 14 | OH | S)-$CH_2CH(OCOC_{13}H_{27})C_{13}H_{27}$ | $-C_{13}H_{27}$ |
| 15 | OMe | $-C_{13}H_{27}$ | $-C_{13}H_{27}$ |
| 16 | OMe | (R)-$CH_2CH(OCOC_5H_{11})C_{11}H_{23}$ | $-C_5H_{11}$ |
| 17 | OMe | (S)-$CH_2CH(OCOC_5H_{11})C_{11}H_{23}$ | $-C_5H_{11}$ |
| 18 | OMe | (R)-$CH_2CH(OCOC_9H_{19})C_{11}H_{23}$ | $-C_9H_{19}$ |
| 19 | OMe | (S)-$CH_2CH(OCOC_9H_{19})C_{11}H_{23}$ | $-C_9H_{19}$ |
| 20 | OMe | (R)-$CH_2CH(OCOC_{13}H_{27})C_{13}H_{27}$( | $-C_{13}H_{27}-$ |
| 21 | OMe | S)-$CH_2CH(OCOC_{13}H_{27})C_{13}H_{27}$ | $C_{13}H_{27}$ |

## Table 24

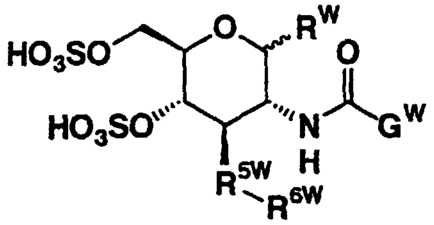

(W-12)

| No. | $R^W$ | $—G^W$ | $—R^{5W}—R^{6W}$ |
|---|---|---|---|
| 1 | H | $-C_{13}H_{27}$ | $-C_{13}H_{27}$ |
| 2 | H | (R)-$CH_2CH(OCOC_5H_{11})C_{11}H_{23}$ | $-C_5H_{11}$ |
| 3 | H | (S)-$CH_2CH(OCOC_5H_{11})C_{11}H_{23}$ | $-C_5H_{11}$ |
| 4 | H | (R)-$CH_2CH(OCOC_9H_{19})C_{11}H_{23}$ | $-C_9H_{19}$ |
| 5 | H | (S)-$CH_2CH(OCOC_9H_{19})C_{11}H_{23}$ | $-C_9H_{19}$ |
| 6 | H | (R)-$CH_2CH(OCOC_{13}H_{27})C_{13}H_{27}$ | $-C_{13}H_{27}$ |
| 7 | H | (S)-$CH_2CH(OCOC_{13}H_{27})C_{13}H_{27}$ | $-C_{13}H_{27}$ |
| 8 | OH | $-C_{13}H_{27}$ | $-C_{13}H_{27}$ |
| 9 | OH | (R)-$CH_2CH(OCOC_5H_{11})C_{11}H_{23}$ | $-C_5H_{11}$ |
| 10 | OH | (S)-$CH_2CH(OCOC_5H_{11})C_{11}H_{23}$ | $-C_5H_{11}$ |
| 11 | OH | (R)-$CH_2CH(OCOC_9H_{19})C_{11}H_{23}$ | $-C_9H_{19}$ |
| 12 | OH | (S)-$CH_2CH(OCOC_9H_{19})C_{11}H_{23}$ | $-C_9H_{19}$ |
| 13 | OH | (R)-$CH_2CH(OCOC_{13}H_{27})C_{13}H_{27}$( | $-C_{13}H_{27}$ |
| 14 | OH | S)-$CH_2CH(OCOC_{13}H_{27})C_{13}H_{27}$ | $-C_{13}H_{27}$ |
| 15 | OMe | $-C_{13}H_{27}$ | $-C_{13}H_{27}$ |
| 16 | OMe | (R)-$CH_2CH(OCOC_5H_{11})C_{11}H_{23}$ | $-C_5H_{11}$ |
| 17 | OMe | (S)-$CH_2CH(OCOC_5H_{11})C_{11}H_{23}$ | $-C_5H_{11}$ |
| 18 | OMe | (R)-$CH_2CH(OCOC_9H_{19})C_{11}H_{23}$ | $-C_9H_{19}$ |
| 19 | OMe | (S)-$CH_2CH(OCOC_9H_{19})C_{11}H_{23}$ | $-C_9H_{19}$ |
| 20 | OMe | (R)-$CH_2CH(OCOC_{13}H_{27})C_{13}H_{27}$( | $-C_{13}H_{27}-$ |
| 21 | OMe | S)-$CH_2CH(OCOC_{13}H_{27})C_{13}H_{27}$ | $C_{13}H_{27}$ |

[Salts]

The compounds of formulae (J) and (I) used in the present invention may be converted into corresponding salts by conventional methods. Non-toxic and water-soluble salts are preferable. Appropriate salts include salts of alkali metals (sodium, potassium, etc.), salts of alkaline-earth metals (calcium, magnesium, etc.), ammonium salts and salts of pharmaceutically-acceptable organic amines (tetramethyl ammonium, triethylamine, methylamine, dimethylamine, cyclopentylamine, benzylamine, phenethylamine, piperidine, monoethanolamine, diethanolamine, lysine, arginine, N-methyl-D-glucamine, tris(hydroxymethyl)methylamine, etc.).

The compounds of formulae (J) and (I) used in the present invention or salts thereof may be converted to hydrates by conventional methods.

[The process for the preparation]

The compounds of formulae (J), (I), (I-A), (I-B), (I-C) and (W) and non-toxic salts thereof may be prepared by the methods described in the JP Kokoku No. 4-74359 (i.e. EP 226381), JP Patent 2514070 (i.e. ER 288888), JP Kokai Sho 63-297357, the methods described in the examples, the same methods as them or known methods.

The process for the preparation of sodium salt of the compound of formula (I-a) is described in example 2, 3, 5 and 6 of JP Kokai Hei 6-41175 (i.e. EP 553786), and in example 4 the process for the preparation of tris(hydroxymethyl)methylammonium salt is described in detail, by the same method or by known methods some kinds of salts of the compounds of formulae (J), (I) or (W) are prepared.

[Toxicity]

The toxicity of the compounds of formulae (J), (I) and (W) used in the present invention is very low and they are safe enough for pharmaceutical use. For instance, the $LD_{50}$ value of 2-deoxy-2-[3S-(9-phenylnonanoyloxy)tetradecanoyl]amino-3-O-(9-phenylnonanoyl)-4-O-sulfo-D-glucopyranose sodium salt (compound No. 1) described hereinafter) is 60 ~ 70 mg/kg by intraveneous administration for SD female/male rats.

Industrial Applicability

The compounds of formulae (J), (I) and (W) used in the present invention are applicable for the prevention and / or treatment of HIV infectious diseases (AIDS).

For the purpose above described, the compounds of the present invention of formulae (J), (I) and (W), non-toxic salts or hydrates thereof may normally be administered usually systemically or topically, orally or parenterally.

The doses to be administered are determined depending upon, for example, age, body weight, symptom, the desired therapeutic effect, the route of administration, and the duration of the treatment. In the human adult, the doses per person are generally in the range of from 1 mg to 1000 mg, by oral administration, up to several times per day, and in the range of from 0.1 mg to 100 mg, by parenteral administration (preferably intravenous administration), up to several times per day, or continuous administration from 1 to 24 hours per day from vein.

As mentioned above, the doses to be used depend upon various conditions. Therefore, there are cases in which doses lower than or greater than the ranges specified above may be used.

The compounds of the present invention of formulae (J), (I) and (W) may be administered in the form of, for example, solid forms for oral administration, liquid forms for oral administration, injections, liniments or suppositories for parenteral administration.

Solid forms for oral administration include compressed tablets, pills, capsules, dispersible powders, and granules, etc. Capsules include hard capsules and soft capsules.

In these solid forms, one or more of the active compound(s) may be admixed with at least one inert diluent (e.g. lactose, mannitol, glucose, hydroxypropyl cellulose, microcrystalline cellulose, starch, polyvinylpyrrolidone or magnesium metasilicate aluminate). The forms are admixed with lubricants (e.g. magnesium stearate), stabilizing agents, disintegrants (e.g. cellulose calcium glycolate),and adjuvants to assist dissolution (e.g. glutamic acid, aspartic acid) according to the methods well known to those skilled in the art.

The tablets or pills may, if desired, be coated with films of gastric or intestinal materials (e.g. sugar, gelatin, hydroxypropyl cellulose, hydroxypropylmethyl cellulose phthalate, etc.). And further, coating may include containment within capsules of absorbable materials such as gelatin.

The solid forms may, if desired, be coated with coating agents (e.g. sugar, gelatin, hydroxypropyl cellulose or hydroxypropylmethyl cellulose phthalate), or be coated with two or more films.

Liquid forms for oral administration include pharmaceutically acceptable aqueous solutions, suspensions and emulsions, syrups and elixirs, etc. In such forms, one or more of the active compound(s) may be dissolved, suspended or emulsified into diluent(s) commonly used in the art (e.g. purified water, ethanol or a mixture thereof). Besides such liquid forms may also comprise wetting agents, suspending agents, emulsifying agents, sweetening agents, flavoring agents, aroma, preservative or buffering agent, etc.

The other compositions for oral administration include sprays comprising one or more active ingredient(s). Sprays may comprise additional substances other than inert diluents, such as stabilizing agents (e.g. sodium sulfate), isotonic buffers (e.g. sodium chloride, sodium citrate or citric acid). For preparation of such sprays, for example, the method described in the United States Patent No. 2,868,691 or 3,095,355 may be used.

Injections for parenteral administration in the present invention include sterile aqueous and / or non-aqueous solutions, suspensions and emulsions. Aqueous solutions and emulsions include, for example, distilled water for injection and physiological salt solutions.

Sterile aqueous and non-aqueous liquid agents, suspensions and emulsions may be mixed for use.

The compositions may comprise preservatives, wetting agents, emulsifying agents, dispersing agents, stabilizers (e.g. lactose), solution adjuvants (e.g. glutamic acid, aspartic acid).

These are sterilized by filtration through bacteria retaining filter, combination with germicides or radiation. They may also be manufactured in the form of sterile solid compositions (e.g. freeze-dried compositions), which may be sterilized or be dissolved in sterile distilled water for injection or some other diluent(s) immediately before use.

Injections for parenteral administration include sterile aqueous and / or non-aqueous solutions, suspensions, emulsions. Aqueous solutions and suspensions include, for example, distilled water for injection and physiological salt solutions. Non-aqueous solutions and suspensions include, for example, propylene glycol, polyethylene glycol and olive oil, alcohol e.g. ethanol, polysorbate (registered trademark) and solid forms which are dissolved or suspended into solvent(s) for injection immediately before use. In injections, one or more of the active compound(s) may be dissolved, suspended or emulsified into solvent(s). The solvents may include distilled water for injection, physiological salt solution, vegetable oil, propylene glycol, polyethylene glycol, alcohol, e.g. ethanol, or a mixture thereof.

Other forms for parenteral administration include liquids for external use, ointments and endermic liniments, inhalations, sprays, suppositories and pessaries for vaginal administration which comprise one or more of the active compound(s) and may be prepared by methods known per se.

[Best Mode for Carrying out the Invention]

The following Reference Examples and Examples (Experiment Examples) illustrate the present invention, but do not limit the present invention. The solvents in parentheses show the eluting or developing solvents and the ratios of the solvents used are by volume in chromatographic separations or TLC.

The compounds used in examples 6 and 7 are the compounds shown in the following (1) ~ (18) or in examples 2 ~ 5 (1). In the present specification, $M^+$ means a mixture of salts of $Ca^{2+}/2$, $Na^+$ and $Mg^{2+}$

Compound (1):

2-Deoxy-2-[3S-(9-phenylnonanoyloxy)tetradecanoyl]amino-3-O-(9-phenylnonanoyl)-4-O-sulfo-D-glucopyranose • sodium salt (prepared in the method described in example 2, 3, 5 or 6 of JP Kokai Hei 6-41175 (i.e. EP 553786).)

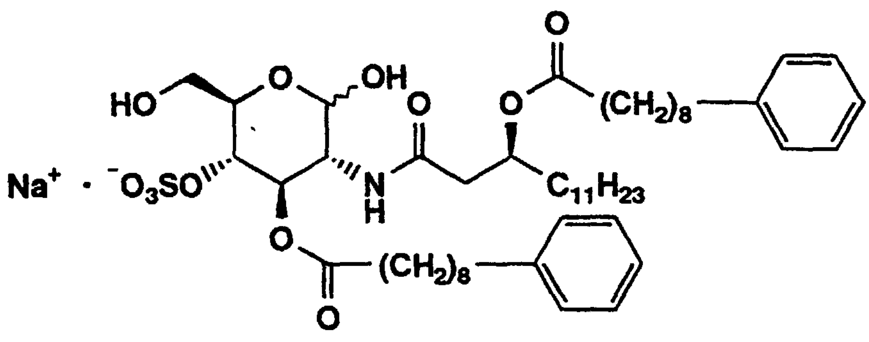

(1)

Compound (2):

A mixture of salts of $Ca^{2+}/2$, $Na^+$ and $Mg^{2+}/2$ of 2-deoxy-2-[3S-(9-phenylnonanoyloxy)-9-phenylnonanoyl]amino-3-O-(9-phenylnonanoyl)-4-O-sulfo-D-glucopyranose (prepared according to the method described in example 1 (r) of JP Kokoku Hei 4-74359 (i.e. EP 226381))

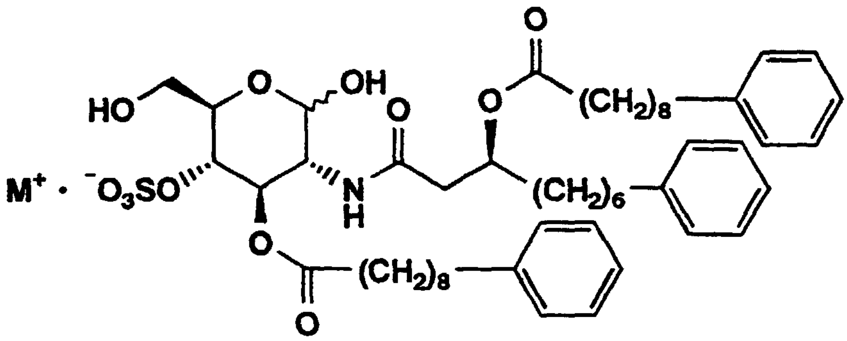

(2)

Compound (3):

A mixture of salts of $Ca^{2+}/2$, $Na^+$ and $Mg^{2+}/2$ of 2-deoxy-2-[3S-[8-(4-methoxyphenyloctanoyloxy)tetradecanoyl]amino-3-O-[8-(4- methoxyphenyloctanoyl)-4-O-sulfo-D-glucopyranose (prepared according to the method described in example 1 (i) of JP Kokoku Hei 4-74359 (i.e. EP 226381)

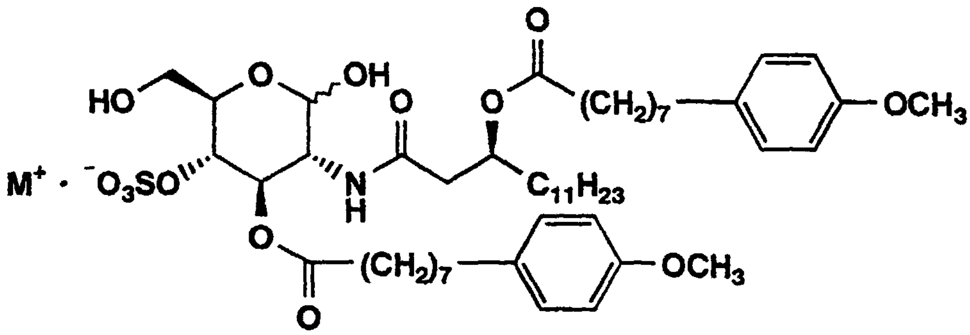

(3)

Compound (4):

A mixture of salts of $Ca^{2+}/2$, $Na^+$ and $Mg^{2+}/2$ of 2-deoxy-2-[3S-[9-(4-chlorophenylnonanoyloxy)tetradecanoyl]amino-3-O-[9-(4-chlorophenylnonanoyl]-4-O-sulfo-D-glucopyranose (prepared according to the method described in example 1 (j) of JP Kokoku Hei 4-74359 (i.e. EP 226381))

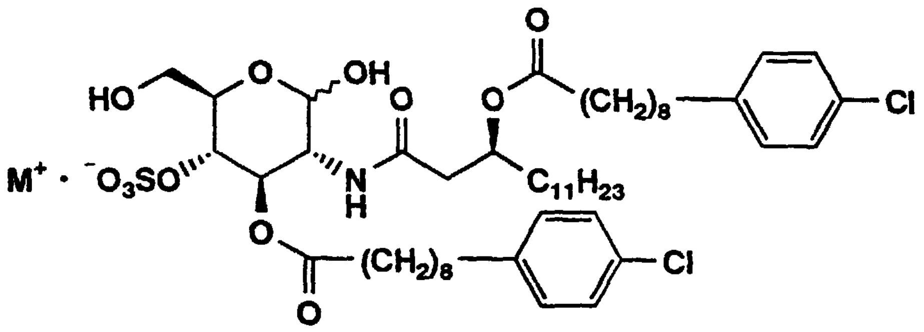

(4)

Compound (5):

A mixture of salts of $Ca^{2+}/2$, $Na^{+}$ and $Mg^{2+}/2$ of 2-deoxy-2-[3S-[5-(4-pentylpheny) pentanoyloxy]tetradecanoyl]amino-3-O-[5-(4-pentylphenyl)pentanoyl]-4-O-sulfo-D-glucopyranose (prepared according to the method described in example 1 (k) of JP Kokoku Hei 4-74359 (i.e. EP 226381))

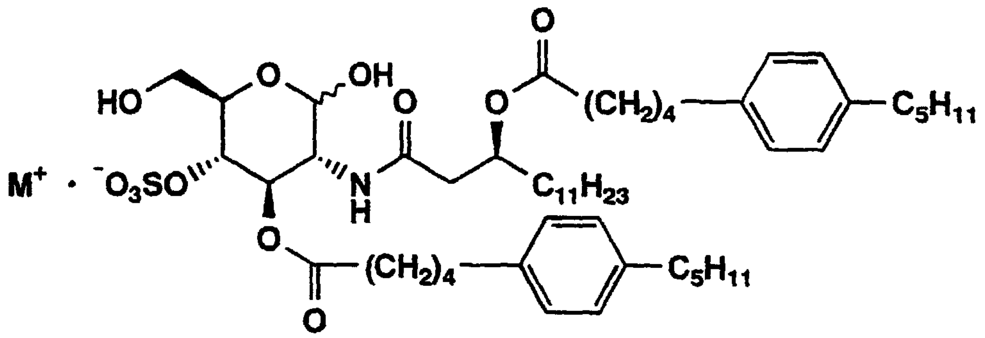

(5)

Compound (6):

A mixture of salts of $Ca^{2+}/2$, $Na^{+}$ and $Mg^{2+}/2$ of 2-deoxy-2-[9-(2-naphthyl)nonanoyl]amino-3-O-[9-(2-naphthyl) nonanoyl]-4-O-sulfo-D-glucopyranose (prepared according to the method described in example 1 (v) of JP Kokoku Hei 4-74359 (i.e. EP 226381))

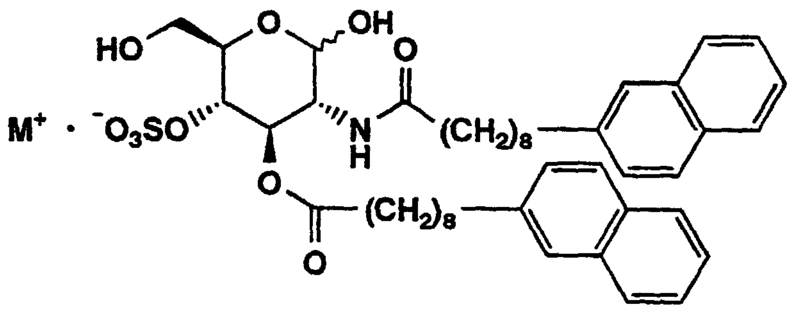

(6)

Compound (7):

A mixture of salts of $Ca^{2+}/2$, $Na^+$ and $Mg^{2+}/2$ of 2-deoxy-2-[3S-[8-(3,5-dichlorophenoxy)octanoyloxy]tetradecanoyl]amino-3-O-[8-(3,5-dichlorophenoxy)octanoyl]-4-O-sulfo-D-glucopyranose (prepared according to the method described in example 1 (n) of JP Kokoku Hei 4-74359 (i.e. EP 226381))

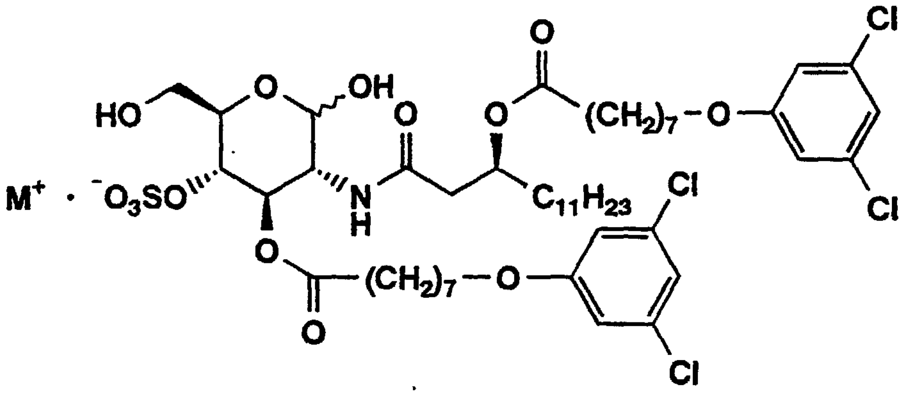

(7)

Compound (8):

A mixture of salts of $Ca^{2+}/2$, $Na^+$ and $Mg^{2+}/2$ of 2-deoxy-2-[3RS-(9-phenylnonanoyloxy)tetradecanoyl]amino-3-O-(9-phenylnonanoyl)-4-O-sulfo-1,5-anhydro-D-xylitol (more polar) (prepared according to the method described in example 1 of JP Patent 2514070 (i.e. EP 288888))

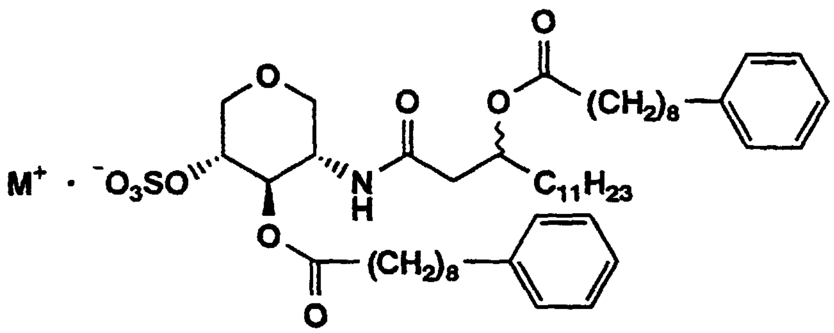

(8)

Compound (9):

A mixture of salts of $Ca^{2+}/2$, $Na^+$ and $Mg^{2+}/2$ of 2-deoxy-2-(3,4-didecyloxybenzoyl)amino-3-O-tetradecanoyl]-4-O-sulfo-D-glucopyranose (prepared according to the method described in example 2 of JP Patent 2514070 (i.e. EP 288888))

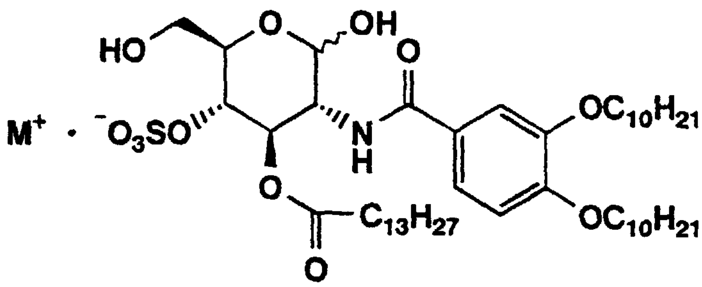

(9)

Compound (10):

A mixture of salts of $Ca^{2+}/2$, $Na^+$ and $Mg^{2+}/2$ of methyl 2-deoxy-2-(3,4-didecyloxybenzoyl) amino-3-O-tetradecanoyl-4-O-sulfo-α-D-glucopyranoside (prepared according to the method described in example 2 (d) of JP Patent 2514070 (i.e. EP 288888))

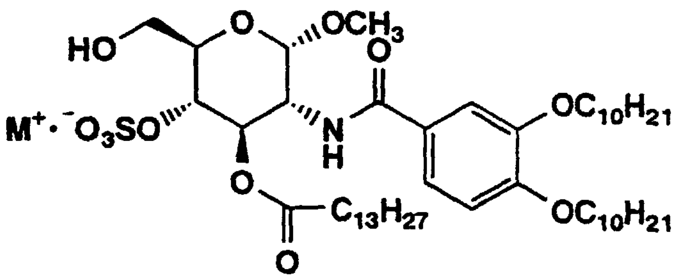

(10)

Compound (11):

A mixture of salts of $Ca^{2+}/2$, $Na^{+}$ and $Mg^{2+}/2$ of methyl 2-deoxy-2-tetradecanoylamino-3-O-[3-(3,4-dioctyloxyphenyl)propanoyl]-4,6-O-disulfo-α -D-glucopyranoside (prepared according to the method described in example 3 (d) of JP Patent 2514070 (i.e. EP 288888))

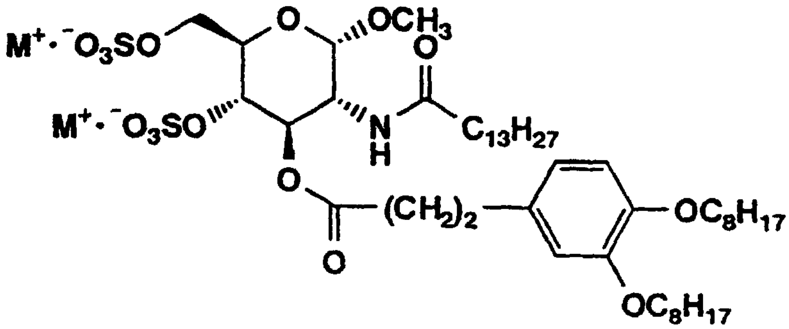

(11)

Compound (12):

A mixture of salts of $Ca^{2+}/2$, $Na^{+}$ and $Mg^{2+}/2$ of 2,6-deoxy-2-[3S-(9-phenylnonanoyloxy)tetradecanoyl]amino-3-O-(9-phenylnonanoyl)-4-O-sulfo-1,5-anhydro-D-glucitol (prepared according to the method described in example 2 of JP Kokoku Hei 4-74359 (i.e. EP 226381))

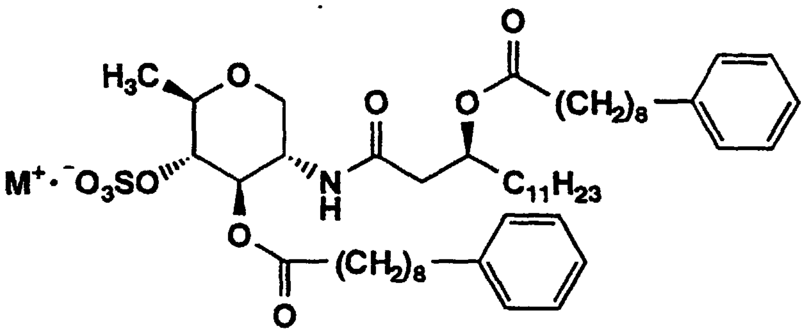

(12)

Compound (13):

A mixture of salts of $Ca^{2+}/2$, $Na^{+}$ and $Mg^{2+}/2$ of 3(syn)-[3RS-(9-phenylnonanoyloxy)tetradecanoyl]amino-2RS(anti)-(9-phenylnonanoyloxy)cyclohexa-1RS-yl-O-sulfonic acid (less polar) (prepared according to the method described in example 1 (c) of JP Kokai Sho 63-297357)

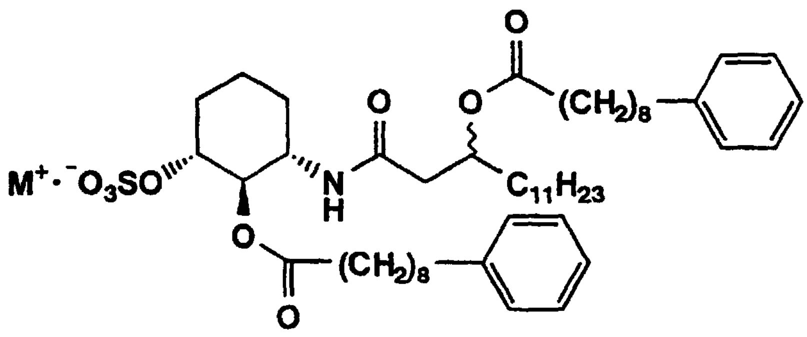

(13)

Compound (14):

2-Deoxy-2-[3S-[3-phenylpropanoyloxy]tetradecanoyl]amino-3-O-[9-phenylnonanoyl]-4-O-sulfo-D-glucopyranose sodium salt (prepared according to the method described in JP Kokoku Hei 4-74359 (i.e. EP 226381))

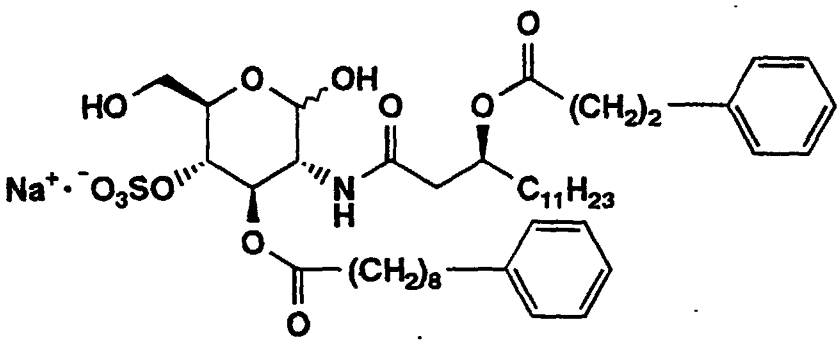

(14)

Compound (15):

2-Deoxy-2-[3S-[9-phenylnonanoyloxy]tetradecanoyl]amino-3-O-[3-phenylpropanoyl]-4-O-sulfo-D-glucopyranose sodium salt (prepared according to the method described in JP Kokoku Hei 4-74359 (i.e. EP

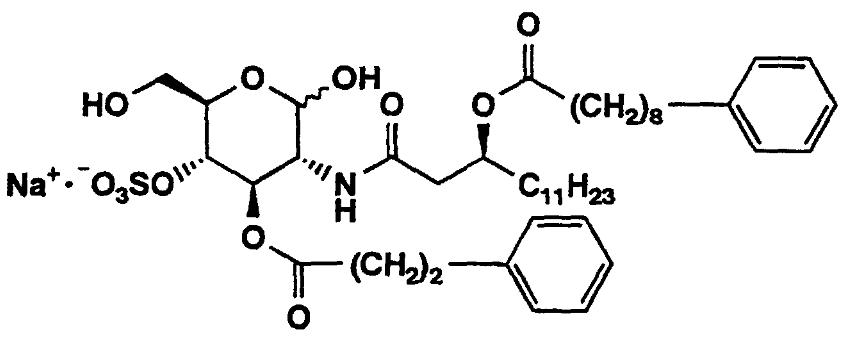

(15)

Compound (16):

2-Deoxy-2-[3S-[9-phenylnonanoyloxy]dodecanoyl]amino-3-O-[9-phenylnonanoyl]-4-O-sulfo-D-glucopyranose sodium salt (prepared according to the method described in JP Kokoku Hei 4-74359 (i.e. EP 226381))

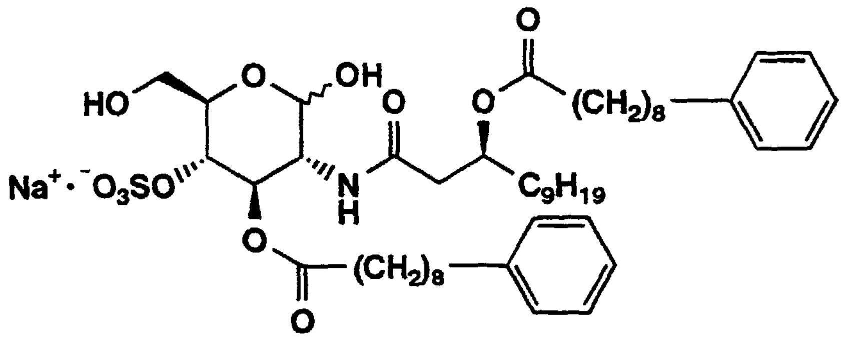

(16)

Compound (17):

2-Deoxy-2-[3S-[9-phenylnonanoyloxy]hexadecanoyl]amino-3-O-[9-phenylnonanoyl]-4-O-sulfo-D-glucopyranose sodium salt (prepared according to the method described in JP Kokoku Hei 4-74359 (i.e. EP 226381))

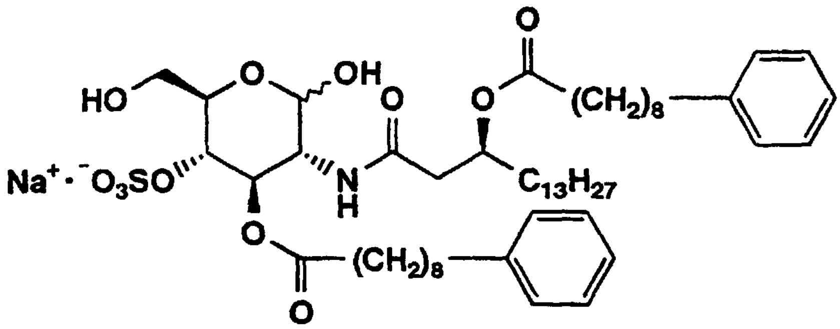

(17)

Compound (18):

2-Deoxy-2-[3R-[9-phenylnonanoyloxy]tetradecanoyl]amino-3-O-[9-phenylnonanoyl]-4-O-sulfo-D-glucopyranose sodium salt (prepared according to the method described in JP Kokoku Hei 4-74359 (i.e. EP 226381))

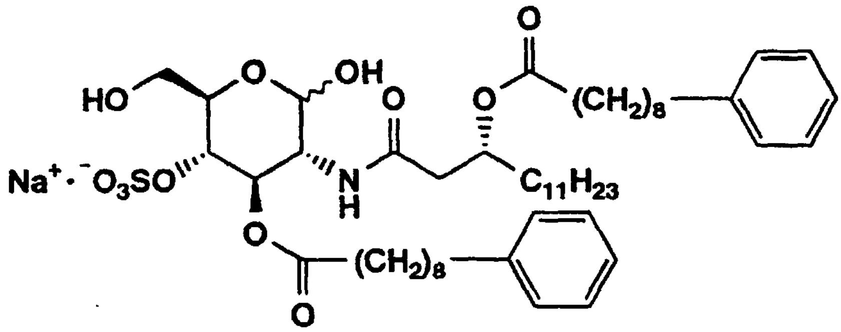

(18)

Reference Example 1:

Benzyl 2-deoxy-2-[3R-(tetradecanoyloxy)tetradecanoyl]amino-3-O-tetradecanoyl-4,6-O-isopropyliden-β-D-glucopyranoside

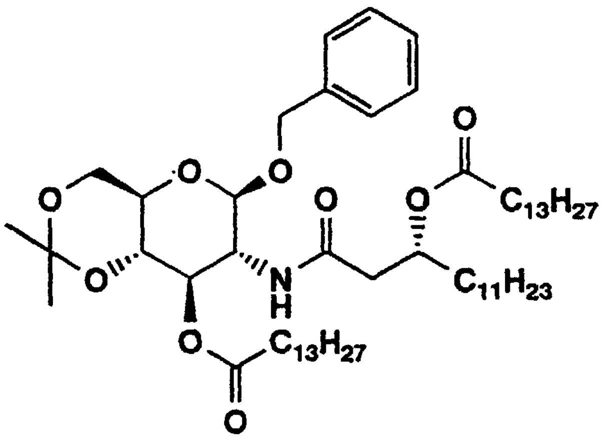

To a solution of benzyl 2-deoxy-2-(3R-hydroxytetradecanoyl)amino-4,6-O-isopropyliden-β-D-glucopyranoside (1.71 g, the compound of reference example 1 in EP 226381) and pyridine (1.03 ml) in dichloromethane (10 ml) under cooling with ice was added myristyl chloride (1.97 ml). The reaction mixture was stirred under cooling with ice for 30 minutes and at room temperature for 2 hours. To the reaction mixture was added methanol (0.1 ml) and the mixture was stirred for 30 minutes. The reaction mixture was diluted with a mixture of hexane and ethyl acetate (1:1) and was washed with 1N hydrochloric acid, a saturated aqueous solution of sodium bicarbonate, water and a saturated aqueous solution of sodium chloride, dried over anhydrous sodium sulfate and was concentrated to give the title compound (4.9 g) having the following physical data.

TLC: Rf 0.88 (hexane : ethyl acetate = 1 : 1).

Reference Example 2:

Benzyl 2-deoxy-2-[3R-(tetradecanoyloxy)tetradecanoyl]amino-3-O-tetradecanoyl-β-D-glucopyranoside

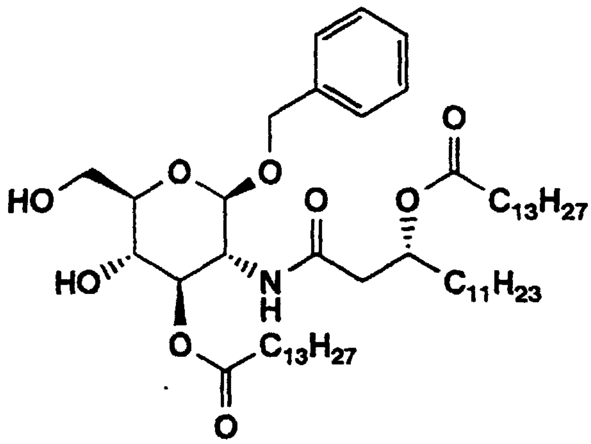

The mixture of the compound prepared in reference example 1 (4.9 g), acetic acid (30 ml), tetrahydrofuran (30 ml) and water (10 ml) was stirred at 50 °C for 18 hours. The reaction mixture was cooled to ambient temperature and thereto was added cold water (100 ml). The crystals that appeared were collected. The crystals were dissolved in dichloromethane and the solution was dried over anhydrous sodium sulfate and was concentrated. The residue was purified by column chromatography on silica get (ethyl acetate : dichloromethane = 5 : 2 → 2 : 1) to give the title compound (2.2. g) having the following physical data.

TLC: Rf 0.11 (hexane : ethyl acetate = 1 : 1).

Reference Example 3:

Benzyl 2-deoxy-2-[3R-(tetradecanoyloxy)tetradecanoyl]amino-3-O-tetradecanoyl-6-O-t-butyldimethylsilyl-β-D-glucopyranoside

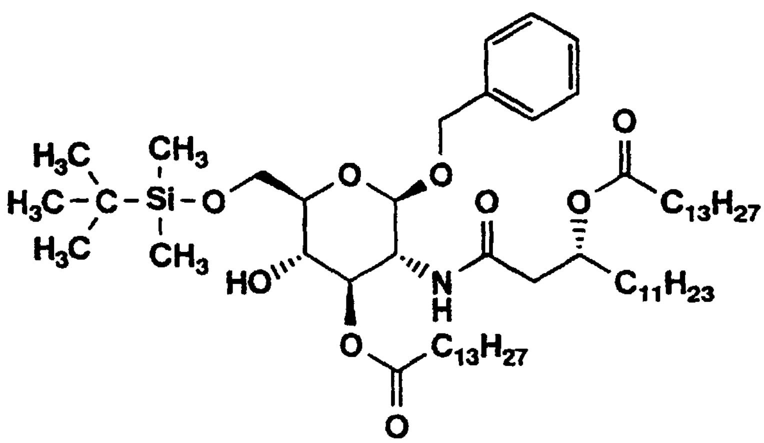

To a solution of the compound prepared in reference example 2 (379 mg) in pyridine (2 ml) was added 4-dimethylaminopyridine (20 mg) and t-butyldimethylsilyl chloride (94 mg). The reaction mixture was stirred at room temperature for 1 hour. To the reaction mixture was added methanol and was diluted with a mixture of hexane and ethyl acetate (1:1). The mixture was washed with 1N hydrochloric acid, a saturated aqueous solution of sodium bicarbonate, water and a saturated aqueous solution of sodium chloride, dried over anhydrous sodium sulfate and was concentrated. The residue was purified by column chromatography on silica gel (ethyl acetate : dichloromethane = 19 : 1 → 9 : 1) to give the title compound (398 mg) having the following physical data.

TLC: Rf 0.26 (ethyl acetate : dichloromethane = 19 : 1).

Reference Example 4:

2-Deoxy-2-[3R-(tetradecanoyloxy)tetradecanoyl]amino-3-O-tetradecanoyl-6-O-t-butyldimethylsilyl-D-glucopyranose

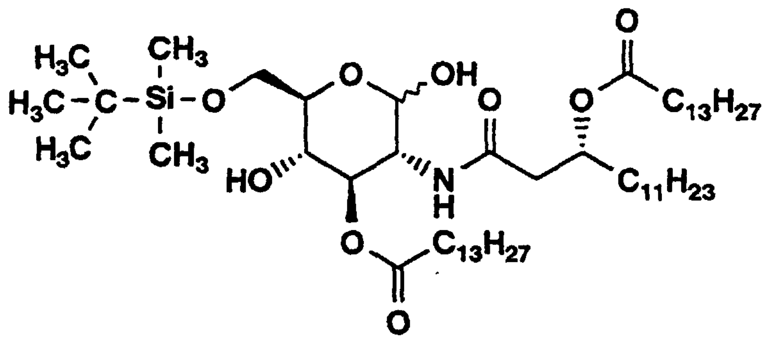

To a solution of the compound prepared in reference example 3 (750 mg) in tetrahydrofuran (6 ml) were added sodium bicarbonate (200 mg) and 10 % palladium-carbon (200 mg). The mixture was stirred under the atmosphere of hydrogen at room temperature overnight. The reaction mixture was filtered and the filtrate was concentrated. The residue was purified by column chromatography on silica gel (ethyl acetate : dichloromethane = 19 : 1 → 9 : 1) to give the title compound (205 mg) having the following physical data.

TLC: Rf 0.26 (ethyl acetate : dichloromethane = 9 : 1).

Reference Example 5:

A mixture of salts of $Ca^{2+}/2$, $Na^{+}$ and $Mg^{2+}/2$ of 2-deoxy-2-[3R-(tetradecanoyloxy) tetradecanoyl]amino-3-O-tetradecanoyl-4-O-sulfo-6-O-t-butyldimethylsilyl-D-glucopyranose

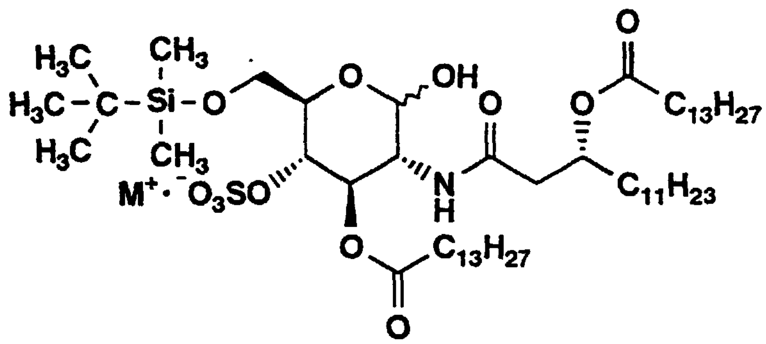

To a solution of the compound prepared in reference example 4 (150 mg) in pyridine (2 ml) was added sulfur trioxide pyridine complex (60 mg). The reaction mixture was stirred at room temperature for 2 hours. The reaction mixture was concentrated. The residue was purified by column chromatography on silica gel (dichloromethane : methanol = 9 : 1) to give the title compound (110 mg) having the following physical data.

TLC: Rf 0.24 (dichloromethane : methanol = 9 : 1).

Example 2: A mixture of salts of $Ca^{2+}/2$, $Na^+$ and $Mg^{2+}/2$ of 2-deoxy-2-[3R-(tetradecanoyloxy)tetradecanoyl]amino-3-O-tetradecanoyl-4-O-sulfo-D-glucopyranose

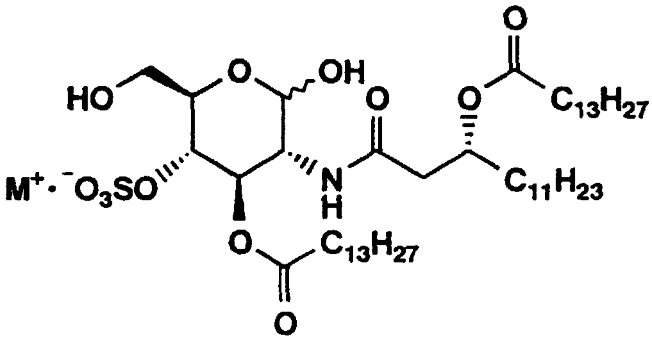

A suspension of the compound prepared in reference example 5 (101 mg) in tetrahydrofuran (2 ml), acetic acid (2 ml) and water (1 ml) was stirred at 50 °C for 20 minutes. The reaction mixture was concentrated. The residue was purified by column chromatography on silica gel (dichloromethane : methanol = 17 : 3) to give the compound of the present invention (66 mg) having the following physical data.

TLC: Rf 0.14 (dichloromethane : methanol = 17 : 3);
IR ($CHCl_3$) : ν3400, 2920, 2850, 1720, 1643, 1453, 1262, 1192, 1110, 1045, 981 $cm^{-1}$.

Example 2 (1) ~ 2 (9)

By the same procedure as described in reference example 1 → reference example 2 → reference example 3 → reference example 4 → reference example 5 → example 2 using a corresponding glucopyranoside derivative in place of benzyl 2-deoxy-2-(3R-hydroxytetradecanoyl)amino-4,6-O-isopropyliden--D-glucopyranoside and a corresponding chloride in place of myristyl chloride, the following compounds of the present invention were obtained.

Example 2 (1): A mixture of salts of $Ca^{2+}/2$, $Na^+$ and $Mg^{2+}/2$ of 2-deoxy-2-octadecanoylamino-3-O-tetradecanoyl-4-O-sulfo-D-glucopyranose

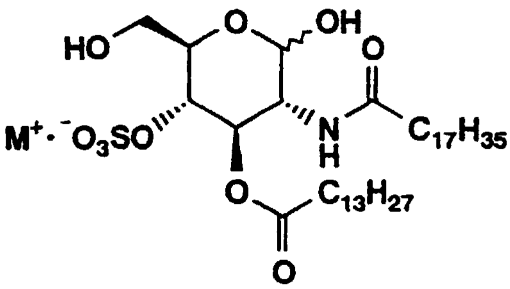

TLC: Rf 0.12 (dichloromethane : methanol = 17 : 3);

IR (KBr): ν 3600-3100, 2920, 2850, 1723, 1660, 1540, 1463, 1240, 1040, 992, 818 $cm^{-1}$.

Example 2 (2): A mixture of salts of $Ca^{2+}/2$, $Na^+$ and $Mg^{2+}/2$ of 2-deoxy-2-[3S-(tetradecanoyloxy)tetradecanoyl]amino-3-O-tetradecanoyl-4-O-sulfo-D-glucopyranose

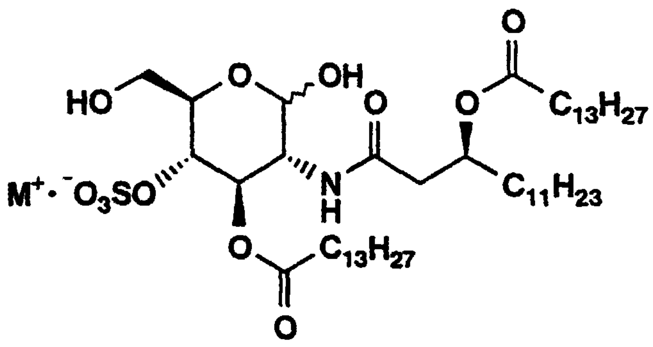

TLC: Rf 0.15 (dichloromethane : methanol = 17 : 3);
IR (KBr): ν 3400, 2925, 2860, 1727, 1658, 1547, 1466, 1228, 1114, 1045, 995, 817, 765, 748, 723, 600 $cm^{-1}$.

Example 2 (3) : A mixture of salts of $Ca^{2+}/2$, $Na^+$ and $Mg^{2+}$ of 2-deoxy-2-[3R-(hexanoyloxy)tetradecanoyl]amino-3-O-hexanoyl-4-O-sulfo-D-glucopyranose

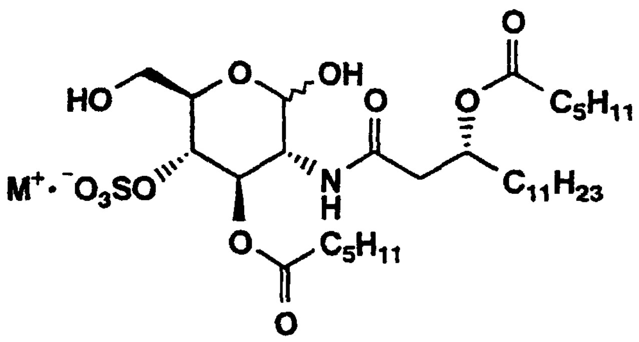

TLC: Rf 0.13 (dichloromethane : methanol = 17 : 3);
IR (KBr) : ν 3400, 2940, 2860, 1733, 1655, 1548, 1462, 1240, 1165, 1114, 1048, 998, 822, 772, 603 $cm^{-1}$.

Example 2 (4) : A mixture of $Ca^{2+}/2$, $Na^+$ and $Mg^{2+}/2$ of 2-deoxy-2-[3R-(decanoyloxy)tetradecanoyl]amino-3-O-decanoyl-4-O-sulfo-D-glucopyranose

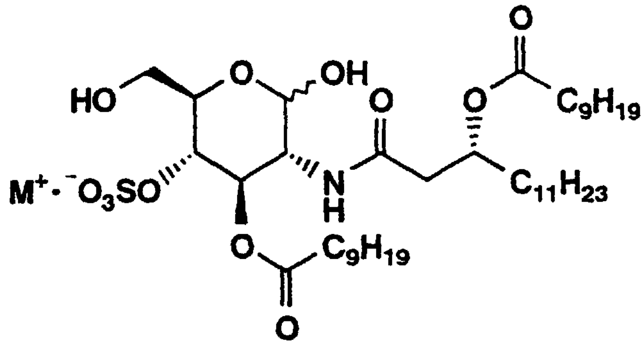

TLC: Rf 0.14 (dichloromethane : methanol = 17 : 3);
IR (KBr) : ν 3400, 2920, 2850, 1725, 1650, 1538, 1460, 1242, 1110, 1042, 995, 820, 765, 600 $cm^{-1}$.

Example 2 (5): A mixture of salts of $Ca^{2+}/2$, $Na^+$ and $Mg^{2+}/2$ of 2-deoxy-2-[3R-(dodecanoyloxy)tetradecanoyl]amino-3-O-dodecanoyl-4-O-sulfo-D-glucopyranose

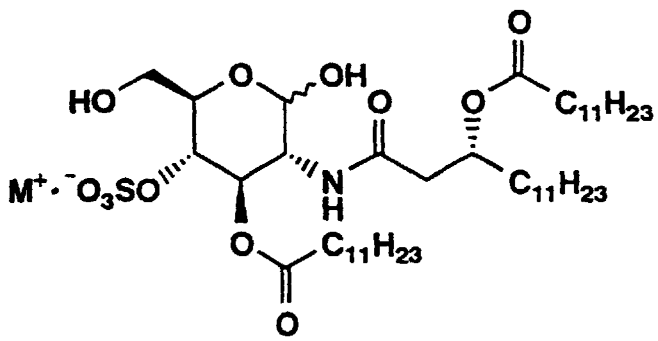

TLC: Rf 0.16 (dichloromethane : methanol = 17 : 3);
IR (KBr): ν 3430, 2930, 2860, 1728, 1655, 1547, 1473, 1250, 1120, 1047, 1000, 973, 825, 607 $cm^{-1}$.

Example 2 (6): A mixture of salts of $Ca^{2+}/2$, $Na^+$ and $Mg^{2+}/2$ of 2-deoxy-2-[3R-(hexadecanoyloxy)tetradecanoyl]amino-3-O-hexadecanoyl-4-O-sulfo-D-glucopyranose

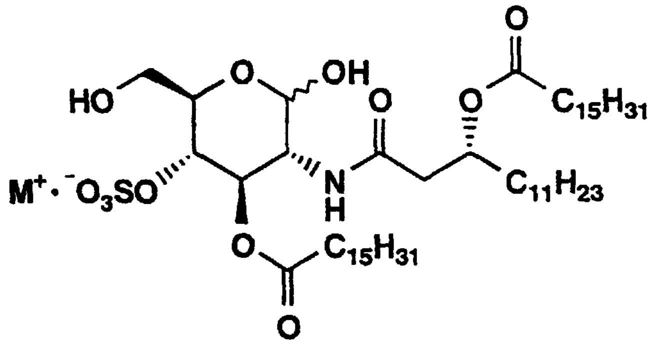

TLC: Rf 0.17 (dichloromethane : methanol = 17 : 3);
IR (KBr): ν 3420, 2920, 2850, 1722, 1653, 1523, 1462, 1246, 1109, 1035, 990, 809, 760, 600 $cm^{-1}$.

Example 2 (7) : A mixture of salts of of $Ca^{2+}/2$, $Na^+$ and $Mg^{2+}/2$ of 2-deoxy-2-[3R-(octadecanoyloxy)tetradecanoyl]amino-3-O-octadecanoyl-4-O-sulfo-D-glucopyranose

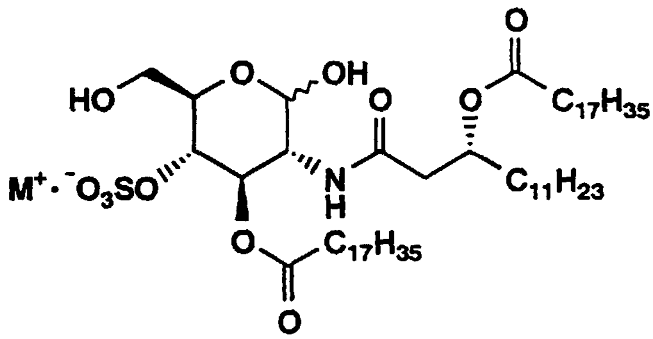

TLC: Rf 0.17 (dichloromethane : methanol = 17 : 3);
IR (KBr) : ν 3415, 2930, 2860, 1728, 1660, 1540, 1472, 1250, 1117, 1042, 998, 817, 768, 725, 608 $cm^{-1}$.

Example 2 (8) : A mixture of salts of of $Ca^{2+}/2$, $Na^+$ and $Mg^{2+}/2$ of 2-deoxy-2-[3R-(icosanoyloxy)tetradecanoyl]amino-3-O-icosanoyl-4-O-sulfo-D-glucopyranose

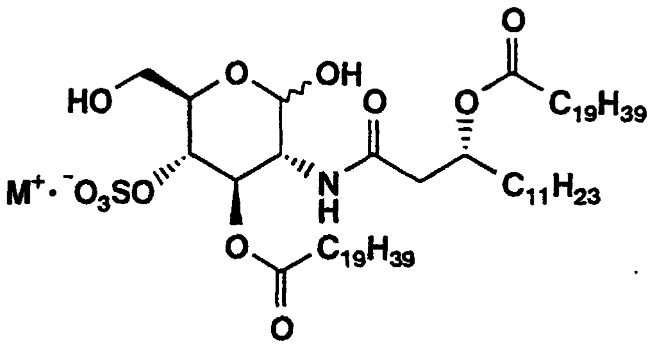

TLC: Rf 0.18 (dichloromethane : methanol = 17 : 3);
IR (KBr) : ν 3400, 2920, 2855, 1722, 1652, 1538, 1460, 1252, 1110, 1045, 1000, 820, 770, 725, 606 $cm^{-1}$.

Example 2 (9) : A mixture of salts of of $Ca^{2+}/2$, $Na^+$ and $Mg^{2+}/2$ of 2-deoxy-2-tetradecanoylamino-3-O-tetradecanoyl-4-O-sulfo-D-glucopyranose

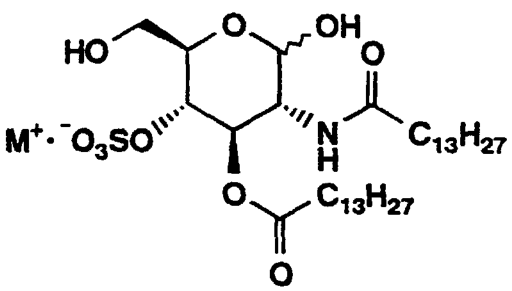

TLC: Rf 0.28 (dichloromethane : methanol = 4 : 1);
IR (KBr): ν 3650-3150, 2920, 2860, 2690, 1725, 1672, 1541, 1468, 1246, 1120, 1033, 992, 820, 744, 722, 592 $cm^{-1}$.

Reference Example 6: 2-deoxy-2-[3S-(tetradecanoyloxy)tetradecanoyl]amino-3-O-tetradecanoyl-D-glucopyranose

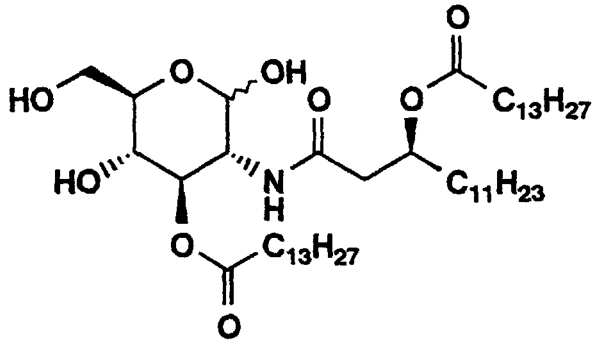

By the same procedure as described in reference example 4 using benzyl 2-deoxy-2-[3S-(tetradecanoyloxy)tetradecanoyl]amino-3-O-tetradecanoyl-β-D-glucopyranoside, the title compound having the following physical data was obtained..

TLC: Rf 0.18 (hexane : ethyl acetate = 1 : 1).

Example 3 : A mixture of salts of $Ca^{2+}/2$, $Na^+$ and $Mg^{2+}/2$ of 2-deoxy-2-[3S-(tetradecanoyloxy)tetradecanoyl]amino-3-O-tetradecanoyl-4-O-sulfo-6-O-sulfo-D-glucopyranose

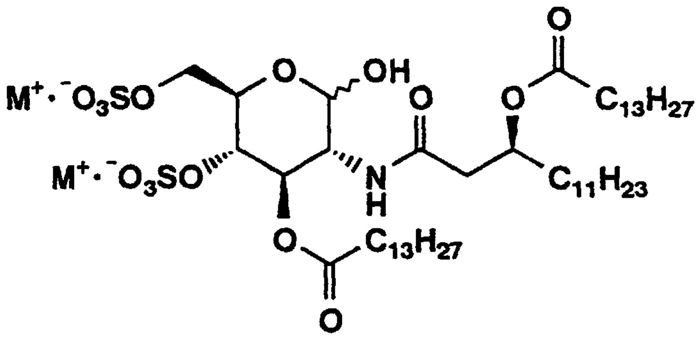

By the same procedure as described in reference example 5 using the compound prepared in reference example 6, the compound of the present invention having the following physical data was obtained.

TLC: Rf 0.23 (dichloromethane : methanol = 5 : 1);
IR (KBr) : ν 3400, 2950, 2870, 1730, 1660, 1640, 1540, 1460, 1390, 1240, 1070, 1010, 780 $cm^{-1}$.

Reference Example 7 : Benzyl 2-deoxy-2-[3S-(tetradecanoyloxy)tetradecanoyl]amino-3-O-tetradecanoyl-6-O-(p- toluenesulfonyl)-β-D-glucopyranoside

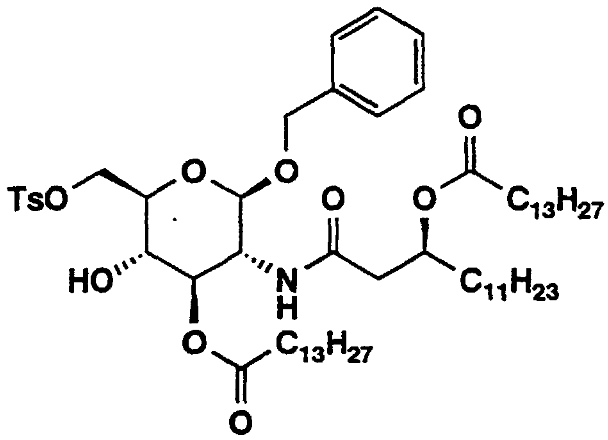

To a solution of benzyl 2-deoxy-2-[3S-(tetradecanoyloxy)tetradecanoyl]amino-3-O-tetradecanoyl-D-glucopyranoside (700 mg) in pyridine (10 ml) were added p-toluenesulfonyl chloride (175 mg) and 4-dimethylaminopyridine (10 mg). The reaction mixture was diluted with ethyl acetate, washed with water and a saturated aqueous solution of sodium chloride, dried over anhydrous magnesium sulfate and was concentrated. The residue was purified by column chromatography on silica gel (hexane : ethyl acetate = 6 : 1 → 3 : 1) to give the title compound (580 mg) having the following physical data.

TLC: Rf 0.75 (hexane : ethyl acetate = 5 : 3).

Reference Example 8: Benzyl 2-deoxy-2-[3S-(tetradecanoyloxy)tetradecanoyl]amino-3-O-tetradecanoyl-6-deoxy-6-iode-D-glucopyranoside

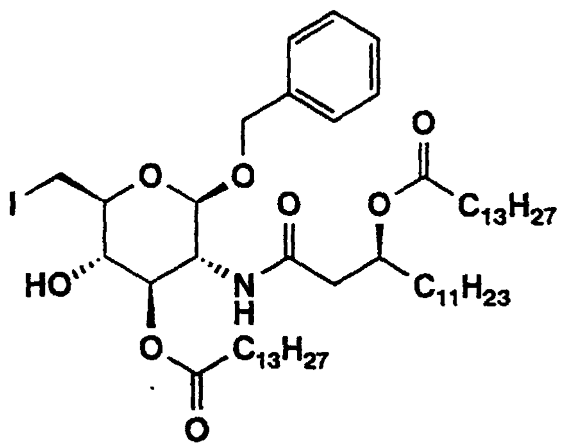

To a solution of the compound prepared in reference example 7 (580 mg) in dimethylformamide (20 ml) was added sodium iodide (500 mg). The reaction mixture was stirred at 50 °C overnight. The reaction mixture was diluted with ethyl acetate, washed with water and a saturated aqueous solution of sodium chloride, dried over anhydrous magnesium sulfate and was concentrated. The residue was purified by column chromatography on silica gel (hexane : ethyl acetate = 3 : 1) to give the title compound (200 mg) having the following physical data.

TLC: Rf 0.45 (hexane : ethyl acetate = 3 : 1).

Reference Example 9: Benzyl 2,6-deoxy-2-[3S-(tetradecanoyloxy)tetradecanoyl]amino-3-O-tetradecanoyl-β-D-glucopyranoside

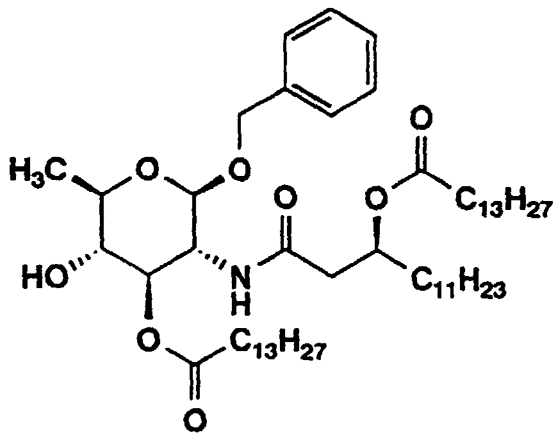

To a solution of the compound prepared in reference example 8 (198 mg) in toluene (50 ml) were added α,α'-azobisisobutyronitrile (AIBN, 10 mg) and tributyltin hydride (n-$Bu_3SnH$, 68 mg). The reaction mixture was stirred at 0 °C in the radiation of mercury lamp for 1.5 hours. The reaction mixture was diluted with ethyl acetate, washed with water and a saturated aqueous solution of sodium chloride, dried over anhydrous magnesium sulfate and was concentrated. The residue was purified by column chromatography on silica gel (hexane : ethyl acetate = 6 : 1) to give the title compound (72 mg) having the following physical data.

TLC: Rf 0.35 (hexane : ethyl acetate = 3 : 1).

Example 4 : A mixture of salts of $Ca^{2+}/2$, $Na^+$ and $Mg^{2+}/2$ of 2,6-deoxy-2-[3S-(tetradecanoyloxy)tetradecanoyl]amino-3-O-tetradecanoyl-4-O-sulfo-D-glucopyranose

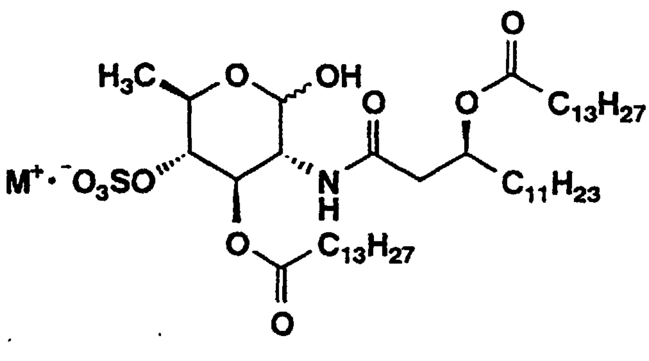

By the same procedure as described in reference example 6 → example 3 using the compound prepared in reference example 9, the compound of the present invention having the following physical data was obtained.

TLC: Rf 0.12 (dichloromethane : methanol = 20 : 1);
IR (KBr) : ν 3450, 2950, 2870, 1730, 1660,, 1540, 1460, 1380, 1260, 1120, 1060, 1010 $cm^{-1}$.

Example 5 and 5 (1)

By the same procedure as described in reference example 1 → reference example 2 → reference example 3 → reference example 5 → example 2 using 2-deoxy-2-(3R-hydroxytetradecanoyl)amino-4,6-O-isopropyliden-1,5-anhydro-D-glucitol (the compound of reference example 1 (d) in EP 226381) and methyl 2-deoxy-2-(3R-hydroxytetradecanoyl)amino-4,6-O-isopropyliden-b-D-glycopyranoside (the compound of reference example 1 (f) in EP 226381).

Example 5: A mixture of salts of $Ca^{2+}$, $Na^+$ and $Mg^{2+}/2$ of 2-deoxy-2-(3R-hydroxytetradecanoyl)amino-3-O-tetradecanoyl-4-O-sulfo-1,5-anhydro-D-glucitol

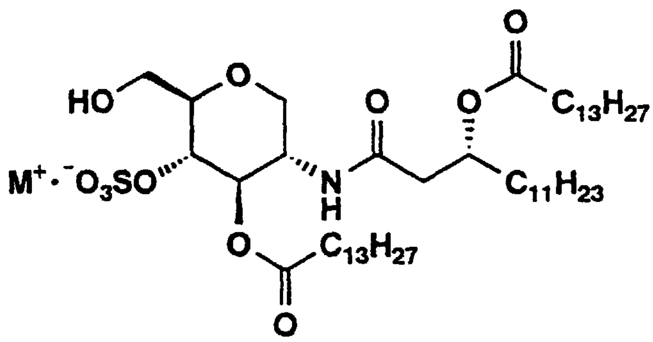

TLC: Rf 0.31 (dichloromethane : methanol = 5 : 1);
IR (KBr) : ν 3410, 2920, 2850, 1730, 1650,, 1540, 1460,, 1240, 1100, 1060, 995, 800 $cm^{-1}$.

Example 5 (1): A mixture of salts of $Ca^{2+}/2$, $Na^+$ and $Mg^{2+}/2$ of methyl 2-deoxy-2-[3R-(tetradecanoyloxy)tetradecanoyl]amino-3-O-tetradecanoyl-4-O-sulfo-β-D-glucopyranoside

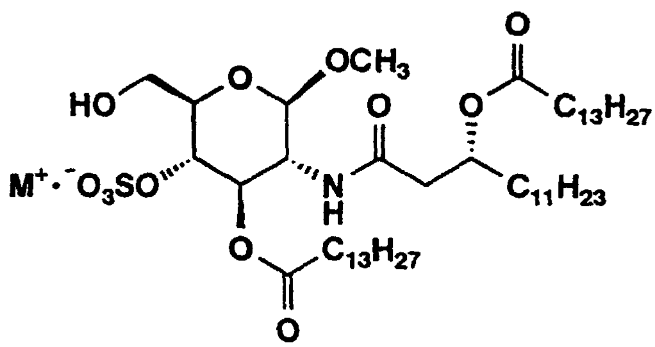

TLC: Rf 0.27 (dichloromethane : methanol = 6 : 1);
IR (KBr): ν 3480-3380, 3070, 2855, 1735, 1650, 1550, 1490, 1455, 1380, 1240, 1040, 1000 $cm^{-1}$.

Examples 6 to 7

As described above, for screening a compound inhibiting the binding of HIV to CXCR4 which is a receptor on T cell, it is a more direct procedure to carry out the screening in an assay system using HIV virus. However, use of HIV virus on a large scale screening is not practical owing to its difficulty of handling. On the other hand, since HIV and

SDF-1 are both bound to CXCR4, it is expectable that there are some common characteristics between the CXCR4 binding sites of both HIV and SDF-1 sides and the SDF-1 and HIV binding sites of CXCR4 side. Therefore, in order to find out a compound inhibiting the adsorption of HIV virus to a cell, i.e., having an activity mechanism different from the existing anti-AIDS medicaments (inhibitors of reverse transcription and inhibitors of proteases), it is possible to utilize an assay system using SDF-1 which is an original ligand for CXCR4, instead of HIV. More particularly, as an example of the screening system of a compound inhibiting the binding of SDF-1 to CXCR4, a system of measuring an effect on transitory increase of Ca ion induced by SDF-1 through CXCR4 is practicable since CXCR4 is a G protein-conjugated seven-times transmembrane-type receptor.

Example 6: Inhibition experiment against induction activity of SDF-1 on transitory increase of Ca ion

Experimental method:

A human T cell expressing CXCR4, for example cells of CCRF-HSB2 (ATCC No. CCL-120.1) were suspended into RPMI-1640 medium, fetal bovine serum (FBS) (10%), Fura-2AM (5μM) (Dojin Kagaku, cat# 343-05401), Probenecid (2.5 mM), and a HEPES buffer (20 mM, pH 7.4) so as to be $5x10^6$ cell/ml, and the suspension was heated at 37°C for 30 minutes under a light-shielded condition. Then, a 4 to 8 fold-diluted Hanks solution (1XHanks), HEPES (20 mM, pH 7.4) and Probenecid (2.5 mM) were added thereto, and the whole was heated for another 30 minutes. After the cells were washed with a solution of 1XHanks, HEPES (20 mM, pH 7.4) and Probenecid (2.5 mM), they were re-suspended into the solution so as to be $2x10^6$ cell/ml. For the T cell on which Fura-2AM had been loaded, the transitory increase of Ca induced by the addition of recombinant SDF-1β dissolved in PBS(-) was detected by use of a suitable Ca detector. In the inhibition experiment, the inhibitory effect of a test compound (10μM) on the transitory increase of Ca induced by the addition of recombinant SDF-1β were measured by adding SDF-1β at an amount so as to be final concentration of, for example, 30 nM at 3 minutes after the addition of the test compound. An inhibition rate (%) was calculated according to the following equation.

$$\text{Inhibition rate} = (Ec - Ea)/Ec \times 100$$

In the equation, each symbol means as follows:

Ec: measured value of the transitory increase of Ca induced by SDF-1β
Ea: measured value of the transitory increase of Ca induced by SDF-1β

when a test compound was added.

Results obtained were shown in Table 25.

Table 25

| Inhibition rate of SDF-1 against activity of inducing transitory increase of Ca ion | |
|---|---|
| Test compound | Inhibition rate (%) |
| Compound (1) | 93 |
| Compound (2) | 96 |
| Compound (3) | 97 |
| Compound (4) | 96 |
| Compound (5) | 97 |
| Compound (6) | 97 |
| Compound (7) | 95 |
| Compound (8) | 94 |
| Compound (9) | 93 |
| Compound (10) | 91 |
| Compound (11) | 92 |

Table 25 (continued)

| Inhibition rate of SDF-1 against activity of inducing transitory increase of Ca ion | |
|---|---|
| Test compound | Inhibition rate (%) |
| Compound (12) | 94 |
| Compound (13) | 86 |
| Compound (14) | 96 |
| Compound (15) | 93 |
| Compound (16) | 92 |
| Compound (17) | 93 |
| Compound (18) | 91 |
| Example 2 (3) | 79 |
| Example 2 (4) | 94 |
| Example 2 (5) | 99 |
| Example 4 | 92 |

Example 7: Inhibition experiment of infection of HIV *in vitro*

Furthermore, the HIV-infection inhibitory activity of each of the compounds which showed effects in Example 6 was confirmed.

Experimental procedure:

Human peripheral blood monocyte was stimulated in RPMI-1640 medium with 10% fetal bovine serum (containing 10% IL2, 50 μg/ml gentamicin, and 0.032% DMSO) in the presence of 1% PHA for 2 days to prepare them for the HIV-infection experiments. One ml of $0.5x10^6$ cell/ml of the human peripheral blood monocyte stimulated with PHA was infected with viruses of HIV IIIB having a 50% infectious dosage ($TCID_{50}$) of $1x10^6$ in the presence of each test compound. After the infection, the HIV viruses were adsorbed to the cells at 37°C for 2 hours in $CO_2$ incubator. Then, the viruses which were not adsorbed were washed out with a phosphate buffer (PBS) and a fresh medium containing each concentration of the test compound was added. After 1 week cultivation, p24 HIV antigen in the supernatant of the culture was detected by ELISA method to evaluate the inhibition of virus growth. During the 1 week cultivation, a fresh medium containing each concentration of the test compound was added on 3rd or 4th day.

The results obtained were shown in Figure 1.

In addition, cytotoxicity was evaluated according to MTS cytotoxicity assay after cultivation of the cells under similar conditions with the exception that no virus was contained. MTS is converted into water-soluble formazan which can be detected by absorption at 490 nM to monitor living cells.

The results obtained were shown in Figure 2.

From the above results, it was found that a glucopyranose derivative of formula (J), (I) or (W), or non-toxic salt thereof has an inhibitory activity on the binding between CXCR4 (SDF-1 receptor) on T cell and SDF-1 and further has an inhibitory activity on the infection of HIV virus, and therefore, is useful for prevention and/or treatment of the infectious diseases (AIDS).

<u>Formulation Example 1</u>

The following components were admixed in a conventional method. The solution was sterilized in a conventional method, placed 1 ml portions into ampoules and freezedried to give 100 ampoules each containing 50 mg of active ingredient.

| | |
|---|---|
| • 2-deoxy-2-[3S-(9-phenylnonanoyloxy)tetradecanoyl]amino-3-O-(9-phenylnonanoyl)-4-O-sulfo-D-glucopyranose sodium salt (compound No. 1) | 5.00 g |
| • 55 % ethanol | 100 ml |

Formulation Example 2

The following components were admixed in a conventional method and punched out to give 100 tablets each containing 5 mg of active ingredient.

| | |
|---|---|
| • 2-deoxy-2-[3S-(9-phenylnonanoyloxy)tetradecanoyl]amino-3-O-(9-phenylnonanoyl)-4-O-sulfo-D-glucopyranose sodium salt (compound No. 1) | 10.0 g |
| • Carboxymethylcellulose calcium | 200 mg |
| • Magnesium stearate | 100 mg |
| • Microcrystaline cellulose | 9.2 g |

**Claims**

1. A medicament for preventing and/or treating HIV infectious diseases comprising, as an active ingredient, a glucopyranose derivative of formula (J):

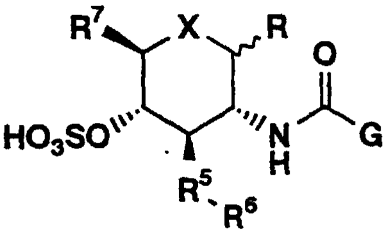

(J)

(wherein X represents an oxygen atom or a methylene group;
R represents a hydrogen atom, a hydroxyl group, or a C1-4 alkoxy group;
G represents:

(1) a group of formula:

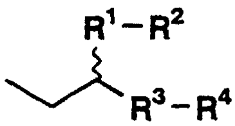

(wherein $R^1$ represents a single bond or a C2-20 oxycarbonylalkylene group;
$R^2$ represents hydrogen atom or a group of formula:

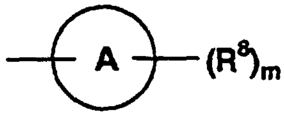

or

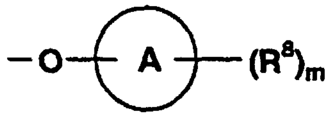

(in each formula, the ring A represents a C5-15 carbon ring; $R^8$ represents a hydrogen atom, a C1-7 alkyl group, a C1-7 alkoxy group, or a halogen atom; and m represents 1, 2 or 3);
$R^3$ represents a C1-20 alkylene group; and
$R^4$ represents a hydrogen atom or a group of formula:

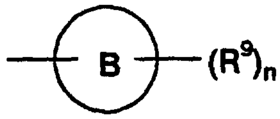

or

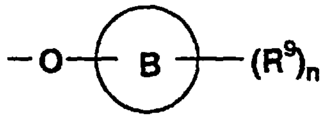

(in each formula, the ring B represents a C5-15 carbon ring; $R^9$ represents a hydrogen atom, a C1-7 alkyl group, a C1-7 alkoxy group, or a halogen atom; and n represents 1, 2 or 3)), or

(2) a group of formula:

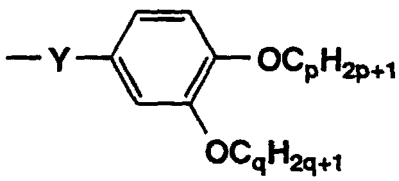

(wherein Y represents a single, bond or a C1-4 alkylene group; and p and q each independently represents an integer of 6 to 12);
$R^5$ represents a C2-20 oxycarbonylalkylene group;
$R^6$ represents a hydrogen atom or a group of formula:

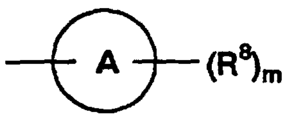

or

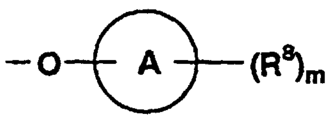

(wherein the ring A, $R^8$ and m have the same meanings as described above), or a combination of $R^5$-$R^6$ represents a group of formula:

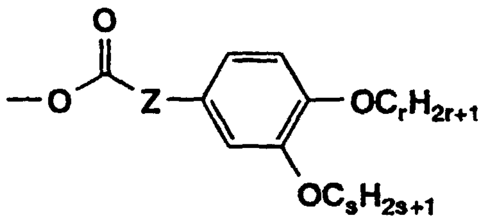

(wherein Z represents a single bond or a C1-4 alkylene group; and r and s each independently represents an integer of 6 to 12); and
$R^7$ represents a hydrogen atom, a methyl group, a hydroxymethyl group, or a sulfoxymethyl group;
with the proviso that

(1) when $R^1$ represents a C2-20 oxycarbonylalkylene group, $R^2$ is bound to the alkyl group in $R^1$,
(2) when $R^5$ represents a C2-20 oxycarbonylalkylene group, $R^6$ is bound to the alkyl group in $R^5$),

or a non-toxic salt thereof;

**2.** A medicament for prevention and/or treatment described in claim 1, comprising as an active ingredient, a glucopyranose derivative of formula (I):

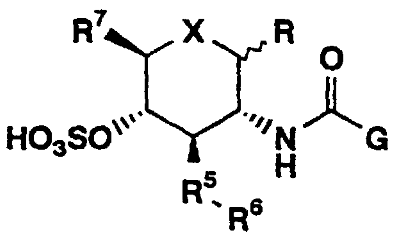

(I)

(wherein X represents an oxygen atom or a methylene group;
R represents a hydrogen atom, a hydroxyl group, or a C1-4 alkoxy group;
G represents:

(1) a group of formula:

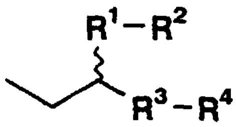

(wherein $R^1$ represents a single bond or a C2-20 oxycarbonylalkylene group;
$R^2$ represents a hydrogen atom or a group of formula:

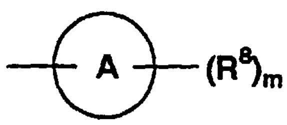

or

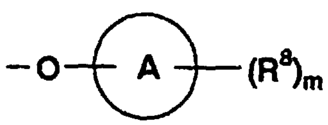

(in each formula, the ring A represents a C5-15 carbon ring; $R^8$ represents hydrogen atom, a C1-7 alkyl group, a C1-7 alkoxy group, or a halogen atom; and m represents 1, 2 or 3);
$R^3$ represents a C1-20 alkylene group; and
$R^4$ represents a hydrogen atom or a group of formula:

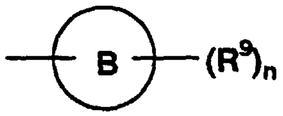

or

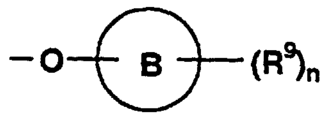

(in each formula, the ring B represents a C5-15 carbon ring; $R^9$ represents a hydrogen atom, a C1-7 alkyl group, a C1-7 alkoxy group, or a halogen atom; and n represents 1, 2 or 3)), or

(2) a group of formula:

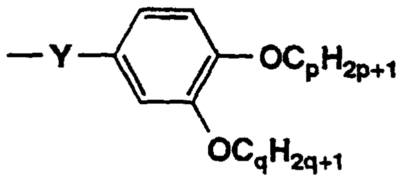

(wherein Y represents a single bond or a C1-4 alkylene group; and p and q each independently represents an integer of 6 to 12);
$R^5$ represents a C2-20 oxycarbonylalkylene group;
$R^6$ represents hydrogen atom or a group of the formula:

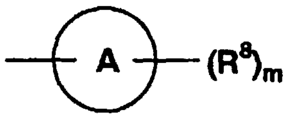

or

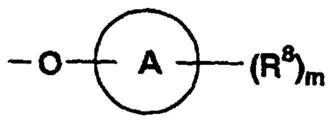

(wherein the ring A, $R^8$ and m have the same meanings as described above), or a combination of $R^5$-$R^6$ represents a group of formula:

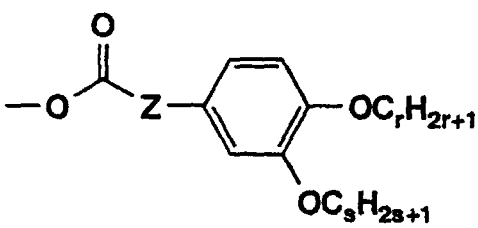

(wherein Z represents a single bond or a C1-4 alkylene group and r and s each independently represents an integer of 6 to 12);
$R^7$ represents a hydrogen atom, a methyl group, a hydroxymethyl group, or a sulfoxymethyl group; with the proviso that

(1) when $R^1$ represents a C2-20 oxycarbonylalkylene group, $R^2$ is bound to the alkyl group in $R^1$,

(2) when $R^5$ represents a C2-20 oxycarbonylalkylene group, $R^6$ is bound to the alkyl group in $R^5$), and

(3) when $R^7$ represents a methyl group, a hydroxymethyl group, or a sulfoxymethyl group, $R^2$, $R^4$ and $R^6$ do not represent hydrogen atoms at the same time),

or a non-toxic salt thereof as an active ingredient.

**3.** A medicament for prevention and / or treatment described in claim 2, comprising as active ingredient, a glucopyranose derivative of formula (I-A)

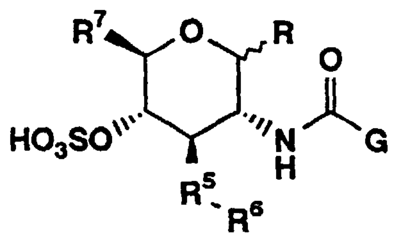

(I-A)

(wherein all symbols are the same meaning as defined in claim 2) or a non-toxic salt thereof.

**4.** A medicament for prevention and / or treatment described in claim 2, comprising as active ingredient, a cyclohexane derivative of formula (I-B)

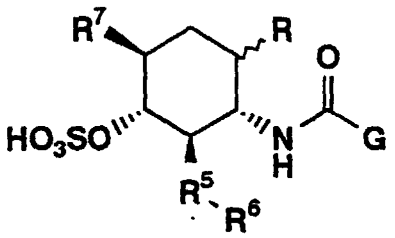

(I-B)

(wherein all symbols are the same meaning as defined in claim 2) or a non-toxic salt thereof.

**5.** A medicament for prevention and / or treatment described in claim 2, comprising as active ingredient, 2-deoxy-2-[3S-(9-phenylnonanoyloxy)tetradecanoyl]amino-3-O-(9-phenylnonanoyl)-4-O-sulfo-D-glucopyranose of formula (I-a)

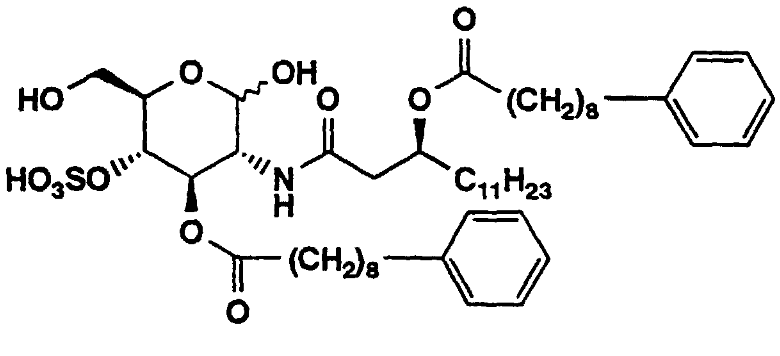

(I-a)

or a non-toxic salt thereof.

**6.** A glucopyranose derivative of formula (W):

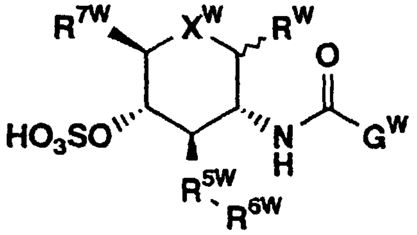

(W)

(wherein $X^W$ represents an oxygen atom or a methylene group;
$R^W$ represents a hydrogen atom, a hydroxyl group, or a C1-4 alkoxy group;
$G^W$ represents:

(1) a group of formula:

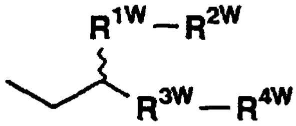

(wherein $R^{1W}$ represents a single bond or a C2-20 oxycarbonylalkylene group;
$R^{2W}$ represents a hydrogen atom;
$R^{3W}$ represents a C1-20 alkylene group;
$R^{4W}$ represents a hydrogen atom;
$R^{5W}$ represents a C2-20 oxycarbonylalkylene group;
$R^{6W}$ represents a hydrogen atom; and
$R^{7W}$ represents a methyl group, a hydroxymethyl group, or a sulfoxymethyl group;
with the proviso that

(1) when $R^{1W}$ represents a C2-20 oxycarbonylalkylene group, $R^{2W}$ is bound to the alkyl group in $R^{1W}$, and
(2) when $R^{5W}$ represents a C2-20 oxycarbonylalkylene group, $R^{6W}$ is bound to the alkyl group in $R^{5W}$),

or a non-toxic salt thereof.

**7.** A compound described in claim 6, which is

(1) 2-deoxy-2-[3R-(tetradecanoyloxy)tetradecanoyl]amino-3-O-tetradecanoyl-4-O-sulfo-D-glusopyranose,
(2) 2-deoxy-2-octadecanoylamino-3-O-tetradecanoyl-4-O-sulfo-D-glucopyranose,
(3) 2-deoxy-2-[3S-(tetradecanoyloxy)tetradecanoyl]amino-3-O-tetradecanoyl-4-O-sulfo-D-glucopyranose,
(4) 2-deoxy-2-[3R-(hexanoyloxy)tetradecanoyl]amino-3-O-hexanoyl-4-O-sulfo-D-glucopyranose,
(5) 2-deoxy-2-[3R-(decanoyloxy)tetradecanoyl]amino-3-O-decanoyl-4-O-sulfo-D-glucopyranose,
(6) 2-deoxy-2-[3R-(dodecanoyloxy)tetradecanoyl]amino-3-O-dodecanoyl-4-O-sulfo-D-glucopyranose,
(7) 2-deoxy-2-[3R-(hexadecanoyloxy)tetradecanoyl]amino-3-O-hexadecanoyl-4-O-sulfo-D-glucopyranose,
(8) 2-deoxy-2-[3R-(octadecanoyloxy)tetradecanoyl]amino-3-O-octadecanoyl-4-O-sulfo-D-glucopyranose,
(9) 2-deoxy-2-[3R-(nonadecanoyloxy)tetradecanoyl]amino-3-O-nonadecanoyl-4-O-sulfo-D-glucopyranose,
(10) 2-deoxy-2-tetradecanoylamino-3-O-tetradecanoyl-4-O-sulfo-D-glucopyranose,
(11) 2-deoxy-2-[3R-(tetradecanoyloxy)tetradecanoyl]amino-3-O-tetradecanoyl-4-O-sulfo-6-O-sulfo-D-glucopyranose,

(12) 2,6-deoxy-2-[3R-(tetradecanoyloxy)tetradecanoyl]amino-3-O-tetradecanoyl-4-O-sulfo-D-glucopyranose,
(13) 2-deoxy-2-(3R-hydroxytetradecanoyl)amino-3-O-tetradecanoyl-4-O-sulfo-1,5-anhydro-D-glycitol????? or
(14) methyl 2-deoxy-2-[3R-(tetradecanoyloxy)tetradecanoyl]amino-3-O-tetradecanoyl-4-O-sulfo-β-D-glucopyranoside.

**8.** A medicament for prevention and / or treatment described in claim 1, comprising as active ingredient, a glucopyranose derivative of formula (W)

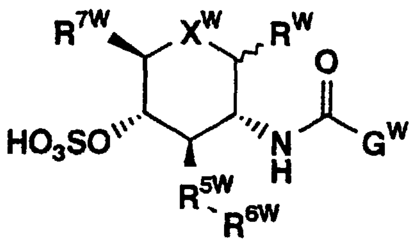

(W)

(wherein all symbols are as defined in claim 6) or a non-toxic salt thereof.

# INTERNATIONAL SEARCH REPORT

International application No.
PCT/JP99/03180

A. CLASSIFICATION OF SUBJECT MATTER
Int.Cl$^6$ A61K31/215, A61K31/70, A61K31/35, C07D309/14, C07C305/20, C07H13/04

According to International Patent Classification (IPC) or to both national classification and IPC

B. FIELDS SEARCHED

Minimum documentation searched (classification system followed by classification symbols)
Int.Cl$^6$ A61K31/215, A61K31/70, A61K31/35, C07D309/14, C07C305/20, C07H13/04

Documentation searched other than minimum documentation to the extent that such documents are included in the fields searched

Electronic data base consulted during the international search (name of data base and, where practicable, search terms used)
CA (STN), REG (STN)

C. DOCUMENTS CONSIDERED TO BE RELEVANT

| Category* | Citation of document, with indication, where appropriate, of the relevant passages | Relevant to claim No. |
|---|---|---|
| P, Y | WO, 9918975, A1 (University of the Ryuukyuu), 22 April, 1999 (22. 04. 99) & AU, 9894582, A1 | 1-8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 6 September, 1999 (06. 09. 99) | 14 September, 1999 (14. 09. 99) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP99/03180

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

Claims 6 and 7 disclose glucopyranose derivatives or nontoxic salts thereof, while claim 8 discloses an invention relating to preventives and/or remedies concerning use of the compounds as set forth in the above claim 6.

However, claims 1 to 5 disclose inventions relating to preventives and/or remedies concerning use of the compounds excluded from the scope as defined in the above claim 6. Such being the case, the two groups of inventions (i.e., a group of inventions as set forth in claims 1 to 5 and another group of inventions as set forth in claims 6 to 8) are not considered as relating to a group of inventions so linked as to form a single general inventive concept (Rule 13.3 of the Regulations under the PCT).

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☐ No protest accompanied the payment of additional search fees.

Form PCT/ISA/210 (continuation of first sheet (1)) (July 1992)